# EUROPEAN PATENT APPLICATION

(11) **EP 0 866 367 A2**
(43) Date of publication of application: **23.09.1998**
(21) Application number: 98104928.1
(22) Date of filing: 18.03.1998
(51) Int. Cl.: G03C 7/12, G03F 7/027, G03F 7/09, G02B 5/20

(54) **Radiation sensitive composition**

(30) Priority: 19.03.1997 JP 84639/97; 16.10.1997 JP 297955/97
(71) Applicant: JSR Corporation, Tokyo 104-0045 (JP)
(72) Inventor: Nagatsuka, Tomio, Yokkaichi, Mie, 510-0957 (JP); Nakamura, Toshio, Yokkaichi, Mie, 510-0957 (JP); Iijima, Takahiro, Mie, 510-0954 (JP); Shimada, Satoshi, Yokkaichi, Mie 510-0034 (JP); Itoh, Yukiko, Nagoya, Aichi 466-0051 (JP); Nemoto, Hiroaki, Yokkaichi, Mie 510-0957 (JP)
(74) Representative: Bühling, Gerhard, Dipl.-Chem.

(57) **Abstract**

A radiation sensitive composition containing (A) at least one colorant selected from an azo pigment, a cyclic diketone pigment and a perylene pigment, (B) an alkali-soluble resin, (C) a polyfunctional monomer and (D) a photopolymerization initiator.

## Description

### Detailed Description of the Invention

This invention relates to a radiation sensitive composition. More specifically, it relates to a radiation sensitive composition advantageously used in the production of a transmission type or reflection type color filter which is used in a color liquid crystal display device, color image pickup tube element and the like.

Color filters used in a color liquid crystal display device, color image pickup tube element and the like are produced by a method (dyeing method) in which a coating film of a photosensitive resin is irradiated with radiation (to be referred to as "exposure" hereinafter) through a photomask, exposed portions thereof are cured and developed, unexposed portions are removed to form a pattern, and then, dyeing is effected; a photolithography method in which a coating film is formed using a composition comprising a photosensitive resin and a colorant dispersed therein, exposed and developed in the same manner as in the dyeing method; and the like. As the colorants for the color filters, a combination of cyan, magenta and yellow complementary colors are used in addition to colorants for three primary colors, i.e., red, green and blue, especially in the case of a color camera tube.

In color filters used in a color liquid crystal display device, color image pickup tube element and the like, pixels having at least two different color tones are arranged, on the surface of a transparent substrate such as glass or the like, in parallel to, or intersectingly with, one another in the case of stripe-shaped pixels, or in both horizontal and transverse directions in the case of square-shaped pixels. The size of pixel is as minute as several tens to several hundreds of micrometers.

To produce a color filter having such fine pixels arranged regularly, there have been heretofore employed a dyeing method in which a photosensitive resin is coated on a substrate, the formed coating film is exposed through a photomask to cause portions exposed to radiation to cure and then developed, unexposed portions thereof are removed to form a pattern, and then, the pattern is dyed; a photolithography method in which a coating film is formed using a composition comprising a photosensitive resin and a colorant such as dye or pigment dissolved or dispersed therein, exposed and developed in the same manner as described above to form a pattern; and the like.

In a color liquid crystal display device, generally, a transparent electrode made from indium oxide or tin oxide is formed on a color filter by vapor deposition or sputtering and further, an alignment film for aligning liquid crystals to a fixed direction is formed on the transparent electrode, in order to drive the liquid crystals. To obtain a high-performance transparent electrode and alignment film, a high temperature of 200°C or higher, preferably 250°C or higher is required when these are formed.

Of color filters produced by the above methods, a color filter using a dye has high transparency to radiation but is insufficient in heat resistance. Therefore, the formation of a transparent electrode and an alignment film must be carried out at a temperature lower than 200°C and in consequence, sufficient performance of the transparent electrode and alignment film can not be ensured. Further, a color filter using a dye has a defect in that it is inferior in light resistance and hence, is not suitable for outdoor use.

Under the circumstances, a pigment which has excellent heat resistance and light resistance is recently used in place of a dye, and in most of color filters currently produced, organic pigments are used.

Color filters are roughly divided into a transmission type color filter which displays images with transmitted light from a backlight installed behind an apparatus; and a reflection type color filter which displays images by reflecting incident light coming from the front side of an apparatus by a light-reflector such as an aluminum foil or the like provided on the rear surface of a transparent substrate installed behind the apparatus.

Reflecting recent improvement in the quality and expansion of application of a color liquid crystal display device, color image pickup tube element or the like, colorants used in the color filters are required to have excellent transparency to radiation, well balance between chroma and lightness, dispersibility in resin components and the like. Also for radiation sensitive compositions, excellent coating uniformity when pixel arrays are formed, solubility in a developer, sensitivity, pattern shape and the like are strictly required.

However, there are a large variety of colorants for use in color filters, and it is extremely difficult to find an appropriate colorant or a combination thereof which is well-balanced in the above characteristic properties.

It is therefore an object of the present invention to provide a novel radiation sensitive composition.

It is another object of the present invention to provide a radiation sensitive composition which is excellent in coating uniformity when pixel arrays are formed, sensitivity and developability.

It is still another object of the present invention to provide a radiation sensitive composition suitable in forming a transmission or reflection type color filter which is excellent in pattern shape, transparency to radiation, balance between chroma and lightness and the like.

Other objects and advantages of the present invention will become apparent from the following description.

According to the present invention, the above objects and advantages of the present invention can be attained by a radiation sensitive composition comprising:
(A) at least one colorant selected from the group consisting of an azo pigment represented by the following formula (1) and a metal complex thereof: 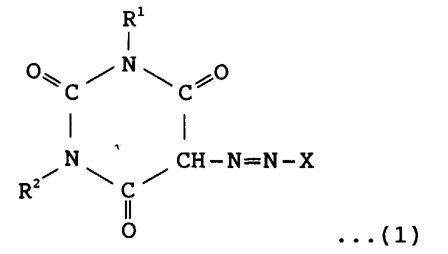
   wherein X is a substituted or unsubstituted monovalent aromatic hydrocarbon group having 6 to 20 carbon atoms, or a group having a substituted or unsubstituted monovalent 5- or 6-membered nitrogen-containing heterocyclic group, and R¹ and R² are independently a hydrogen atom, or a substituted or unsubstituted monovalent hydrocarbon group having 1 to 5 carbon atoms,
   an azo pigment represented by the following formula (2): 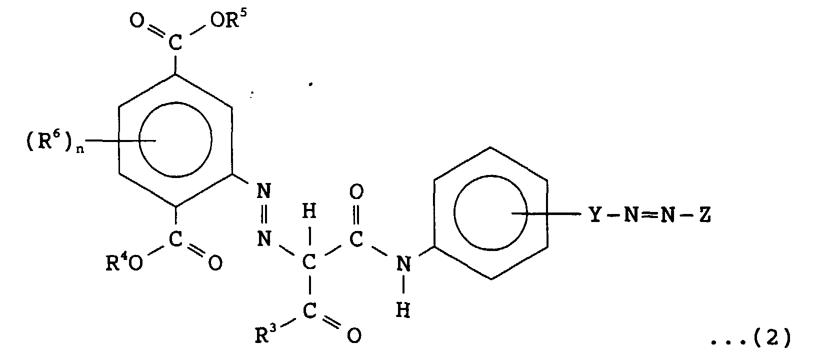
   wherein Y is a divalent bond, Z is a substituted or unsubstituted monovalent aromatic hydrocarbon group having 6 to 20 carbon atoms, or a group having a substituted or unsubstituted monovalent 5- or 6-membered nitrogen-containing heterocyclic group, R³, R⁴ and R⁵ are independently a hydrogen atom, or a substituted or unsubstituted monovalent hydrocarbon group having 1 to 5 carbon atoms, R⁶ is a monovalent substituent group, and n is an integer of 0 to 3,
   a cyclic diketone compound pigment, a perylene pigment represented by the following formula (3): 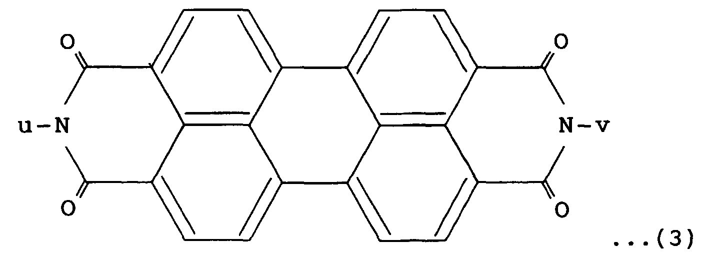
   wherein u and v are independently an alkyl group having 1 to 10 carbon atoms, a substituted or unsubstituted monovalent aromatic hydrocarbon group having 6 to 20 carbon atoms, or a group having a substituted or unsubstituted monovalent 5- or 6-membered nitrogen-containing heterocyclic group,
   and a perylene pigment represented by the following formula (4): 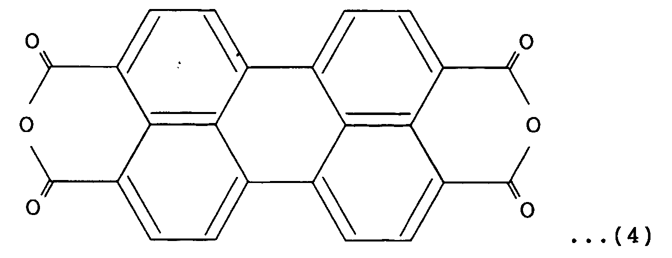
(B) an alkali-soluble resin,
(C) a polyfunctional monomer, and
(D) a photopolymerization initiator.

The present invention will be described in detail hereinafter.

### (A) Colorant

The colorant in the present invention is at least one member selected from the group consisting of an azo pigment represented by the above formula (1) (to be referred to as "pigment (1)" hereinafter), an azo pigment represented by the above formula (2) (to be referred to as "pigment (2)" hereinafter), a cyclic diketone compound pigment (to be referred to as "pigment (3)" hereinafter), a perylene pigment represented by the above formula (3) (to be referred to as "pigment (4)" hereinafter) and a perylene pigment represented by the above formula (4) (to be referred to as "pigment (5)" hereinafter).

In the above formula (1) representing the pigment (1), X is a substituted or unsubstituted monovalent aromatic hydrocarbon group having 6 to 20 carbon atoms, or a group having a substituted or unsubstituted monovalent 5- or 6-membered nitrogen-containing heterocyclic group.

The monovalent aromatic hydrocarbon group having 6 to 20 carbon atoms is, for example, phenyl group, 4-biphenyl group, 1-naphthyl group, 2-naphthyl group, 1-anthranyl group, 2-anthranyl group, 9-anthranyl group, 9-phenanthryl group or the like.

Substituent groups for these groups include, for example, a halogen atom, nitro group, cyano group, -OR⁷, -COR⁸, -COOR⁹, -N(R¹⁰)(R¹¹), -SO₃R¹², -SO₂R¹³, -OCOR¹⁴ (in which R⁷ to R¹² are independently a hydrogen atom, alkyl group having 1 to 5 carbon atoms, aryl group having 6 to 12 carbon atoms or aralkyl group having 7 to 13 carbon atoms, and R¹³ and R¹⁴ are independently an alkyl group having 1 to 5 carbon atoms, aryl group having 6 to 12 carbon atoms or aralkyl group having 7 to 13 carbon atoms) and the like. At least one of these substituent groups may be present at an appropriate position of the compound.

The group having a 5- or 6-membered nitrogen-containing heterocyclic group, represented by X, is, for example, pyrrolyl group, pyrazolyl group, imidazolyl group, triazolyl group, pyridinyl group, pyrimidinyl group, pyrazinyl group, 1,3,5-triazinyl group, oxazolyl group, isooxazolyl group, thiazolyl group, isothiazolyl group, group represented by the following formula (5), (6), (7) or (8), or the like.

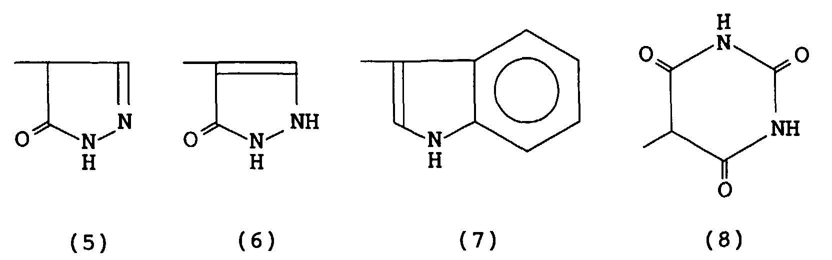

Of substituent groups for the above groups, substituent groups for the nitrogen atom of the group having a nitrogen-containing heterocyclic group include, for example, an alkyl group having 1 to 5 carbon atoms, aryl group having 6 to 12 carbon atoms, aralkyl group having 7 to 13 carbon atoms and the like, and substituent groups for the carbon atom of the group having a nitrogen-containing heterocyclic group include, for example, an alkyl group having 1 to 5 carbon atoms, aryl group having 6 to 12 carbon atoms, aralkyl group having 7 to 13 carbon atoms, and the same groups as the substituent groups for the aromatic hydrocarbon group. At least one of these substituent groups may be present at an appropriate position of the compound.

In the above formula (1), R¹ and R² are independently a hydrogen atom, or a substituted or unsubstituted monovalent hydrocarbon group having 1 to 5 carbon atoms.

The monovalent hydrocarbon group having 1 to 5 carbon atoms, represented by R¹ and R² is, for example, methyl group, ethyl group, n-propyl group, i-propyl group, n-butyl group, i-butyl group, sec-butyl group, t-butyl group, n-pentyl group or the like.

Substituent groups for the hydrocarbon group represented by R¹ and R² are the same as those listed for the aromatic hydrocarbon group represented by X.

In the above formula (1), when at least one of R¹ and R² is a hydrogen atom, a keto-enol tautomer structure can be established between a nitrogen atom bonded to the hydrogen atom and any one of carbonyl groups adjacent thereto.

Specific examples of the pigment (1) include compounds represented by the following formulas (9) to (30), 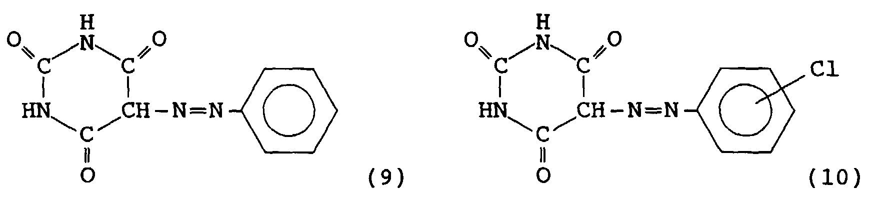 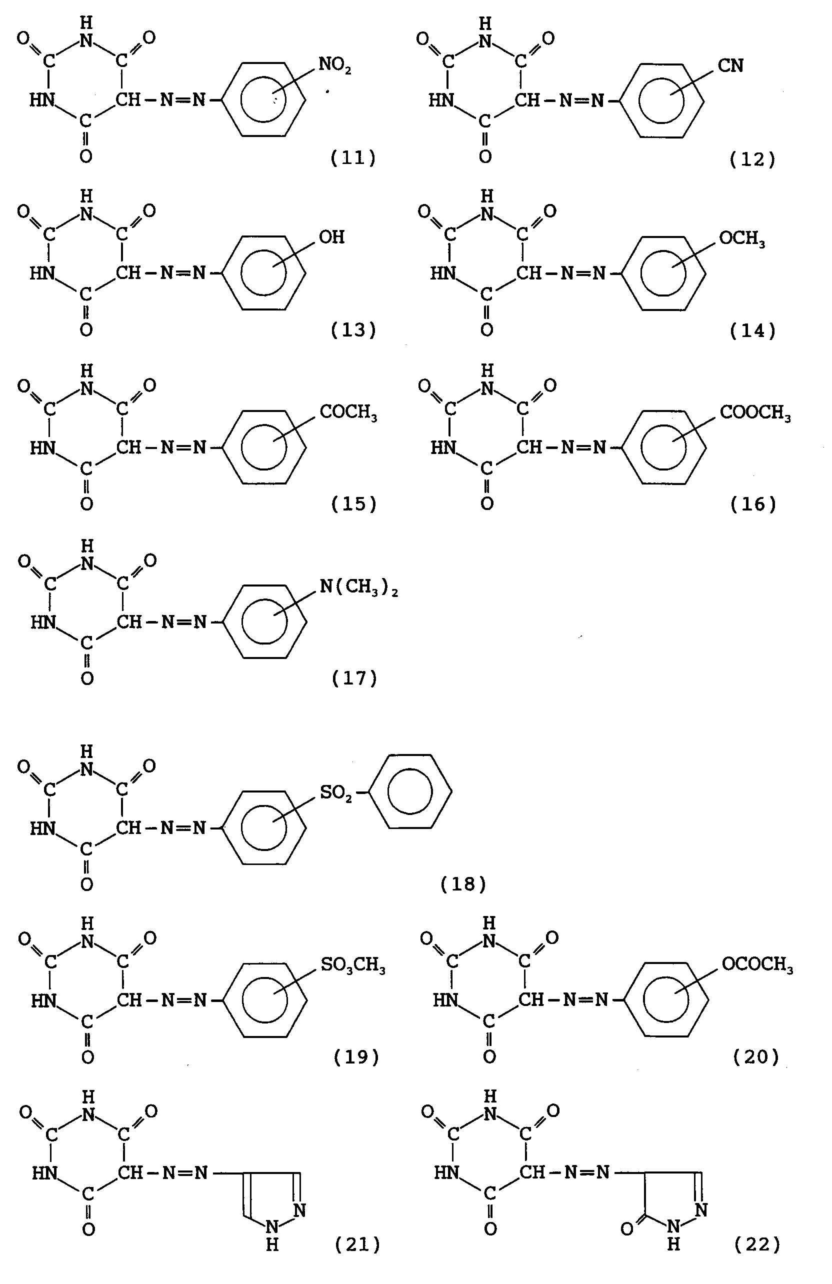 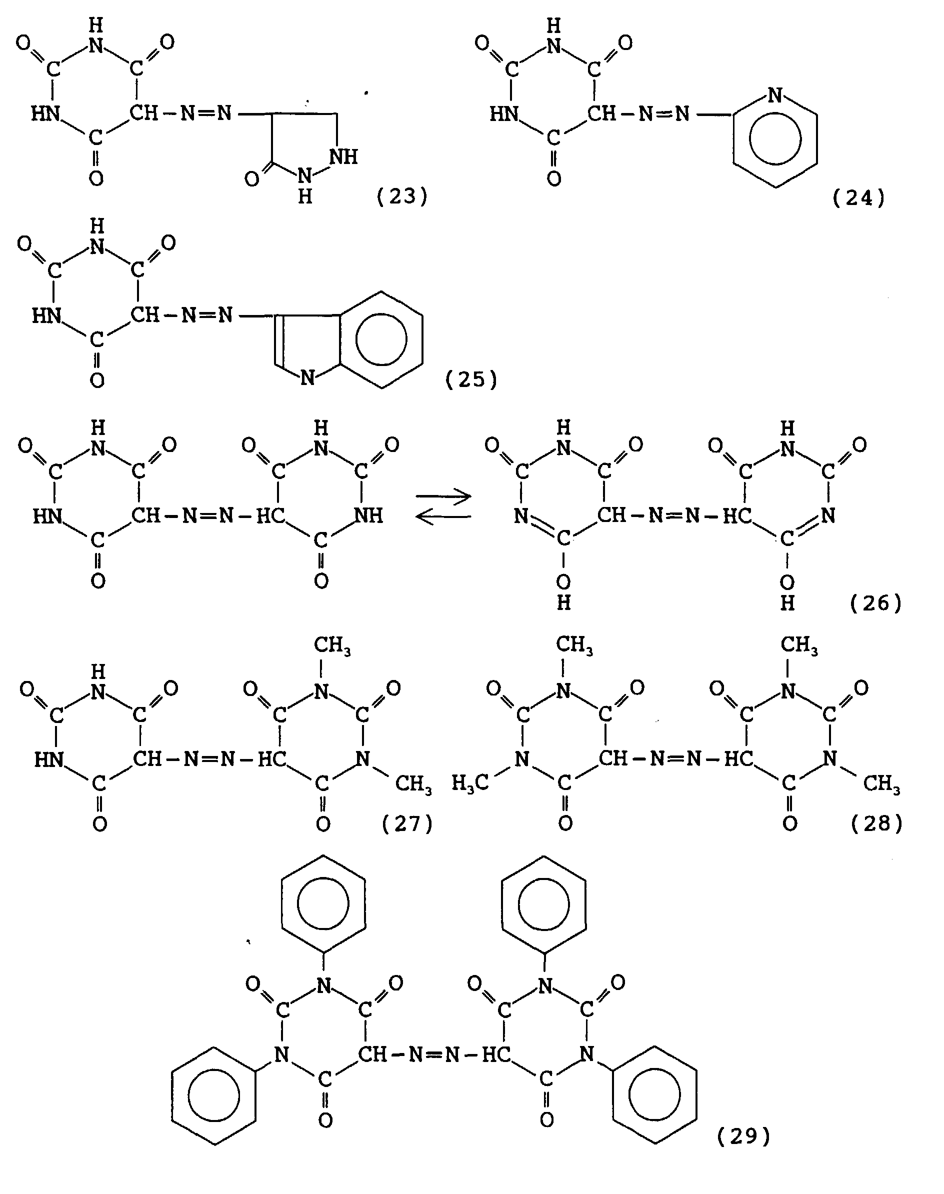 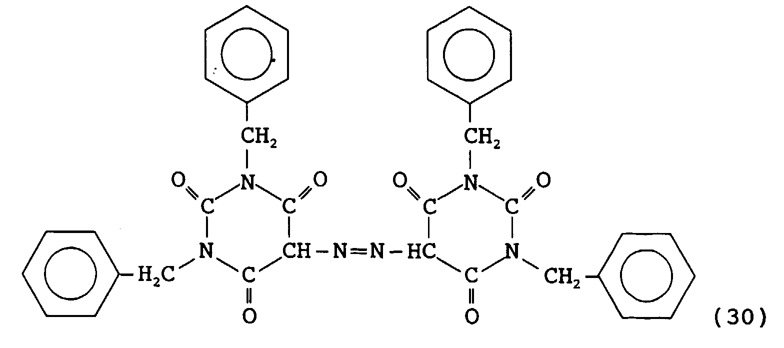 metal complexes thereof and the like.

Metals forming the above metal complexes include, for example, iron, cobalt, nickel, chromium, copper, magnesium and the like, out of which nickel is particularly preferred.

The pigment (1) in the present invention is preferably a metal complex of a compound of the general formula (1) in which R¹ and R² are both a hydrogen atom, more preferably a metal complex of a compound represented by the formula (26), particularly preferably a nickel complex of a compound represented by the formula (26).

The nickel complex of the compound represented by the formula (26) corresponds to C.I. Pigment Yellow 150 according to the Color Index (C.I.) (published by The Society of Dyers and Colourists, the same shall apply hereinafter).

In the above formula (2) representing the pigment (2), Y is a divalent linkage group, and examples thereof include a single bond, an alkylene group having 1 to 5 carbon atoms, arylene group having 6 to 12 carbon atoms, aralkylene group having 7 to 13 carbon atoms, -O-(CH₂)ᵢ-, -S-(CH₂)ᵢ-, -CO-(CH₂)ᵢ-, -COO-(CH₂)ᵢ-, -OCO-(CH₂)ᵢ-, -SO₂-(CH₂)ᵢ-, -NH-(CH₂)ᵢ-, -NHCO-(CH₂)ᵢ- and 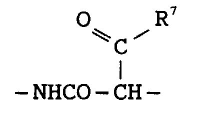 (in which i is an integer of 1 to 5, and R⁷ is a hydrogen atom, or a substituted or unsubstituted monovalent hydrocarbon group having 1 to 5 carbon atoms), and the like.

In the above formula (2), Z is a substituted or unsubstituted monovalent aromatic hydrocarbon group having 6 to 20 carbon atoms, or a group having a substituted or unsubstituted monovalent 5- or 6-membered nitrogen-containing heterocyclic group.

The monovalent aromatic hydrocarbon group having 1 to 20 carbon atoms, represented by Z, is, for example, phenyl group, 4-biphenyl group, 1-naphthyl group, 2-naphthyl group, 1-anthranyl group, 2-anthranyl group, 9-anthranyl group, 9-phenanthryl group or the like.

Substituent groups for the above aromatic hydrocarbon group represented by Z are the same as those listed for the monovalent aromatic hydrocarbon group having 6 to 20 carbon atoms, represented by X in the above formula (1).

The group having a 5- or 6-membered nitrogen-containing heterocyclic group, represented by Z, is, for example, pyrrolyl group, pyrazolyl group, imidazolyl group, triazolyl group, pyridinyl group, pyrimidinyl group, pyrazinyl group, 1,3,5-triazinyl group, oxazolyl group, isooxazolyl group, thiazolyl group, isothiazolyl group, a group represented by the above formula (5), (6), (7) or (8), or the like.

Substituent groups for the above group having a nitrogen-containing heterocyclic group, represented by Z, are the same as those listed for the group having a nitrogen-containing heterocyclic group, represented by X in the above formula (1).

In the above formula (2), R³, R⁴ and R⁵ are independently a hydrogen atom, or a substituted or unsubstituted monovalent hydrocarbon group having 1 to 5 carbon atoms, R⁶ is a monovalent substituent group, and n is an integer of 0 to 3.

The monovalent hydrocarbon group having 1 to 5 carbon atoms, represented by R³ to R⁵, is, for example, methyl group, ethyl group, n-propyl group, i-propyl group, n-butyl group, i-butyl group, sec-butyl group, t-butyl group, n-pentyl group or the like.

Substituent groups for the above hydrocarbon group and examples of the monovalent substituent group represented by R⁶ are the same as those listed for the above aromatic hydrocarbon group represented by Z. When a plurality of R⁵'s are present in the above formula (2), R⁶'s may be the same or different.

Specific examples of the pigment (2) include compounds represented by the following formulas (31) to (42) 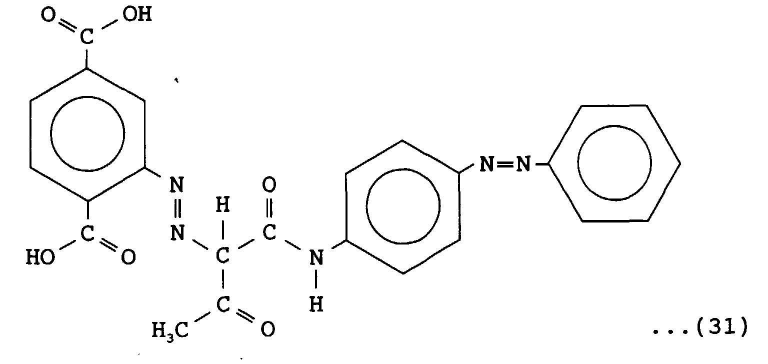 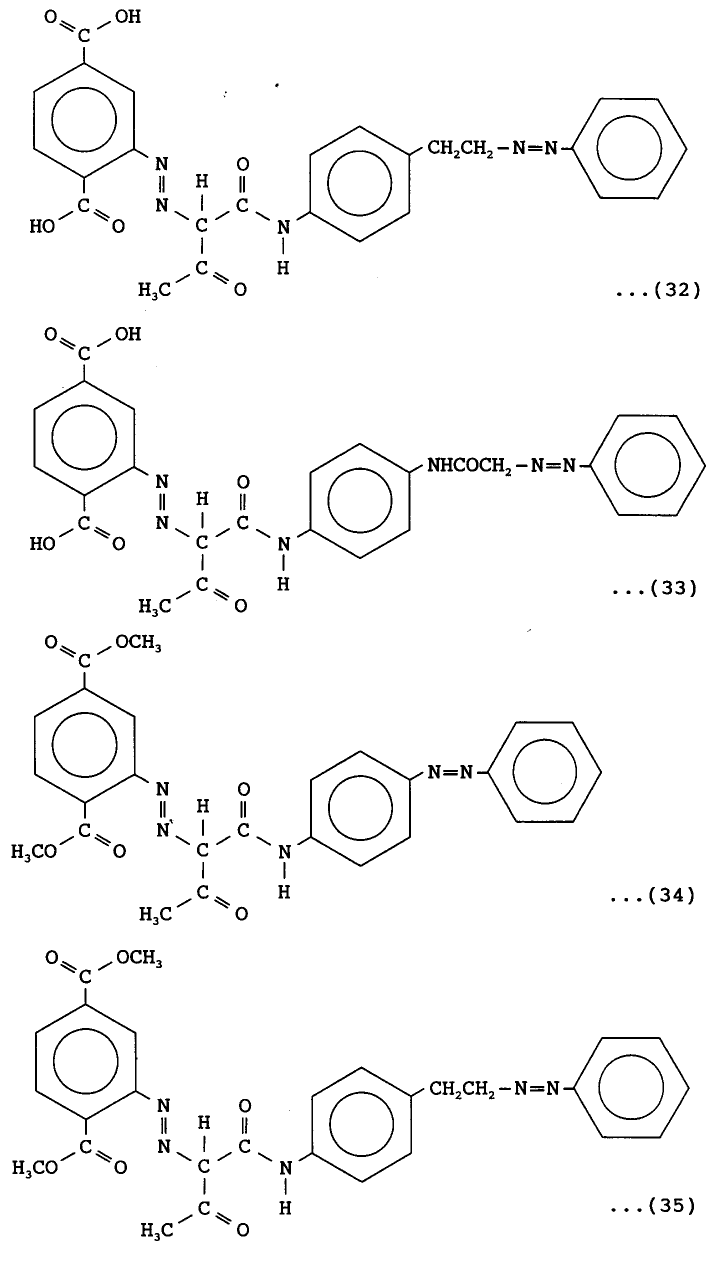 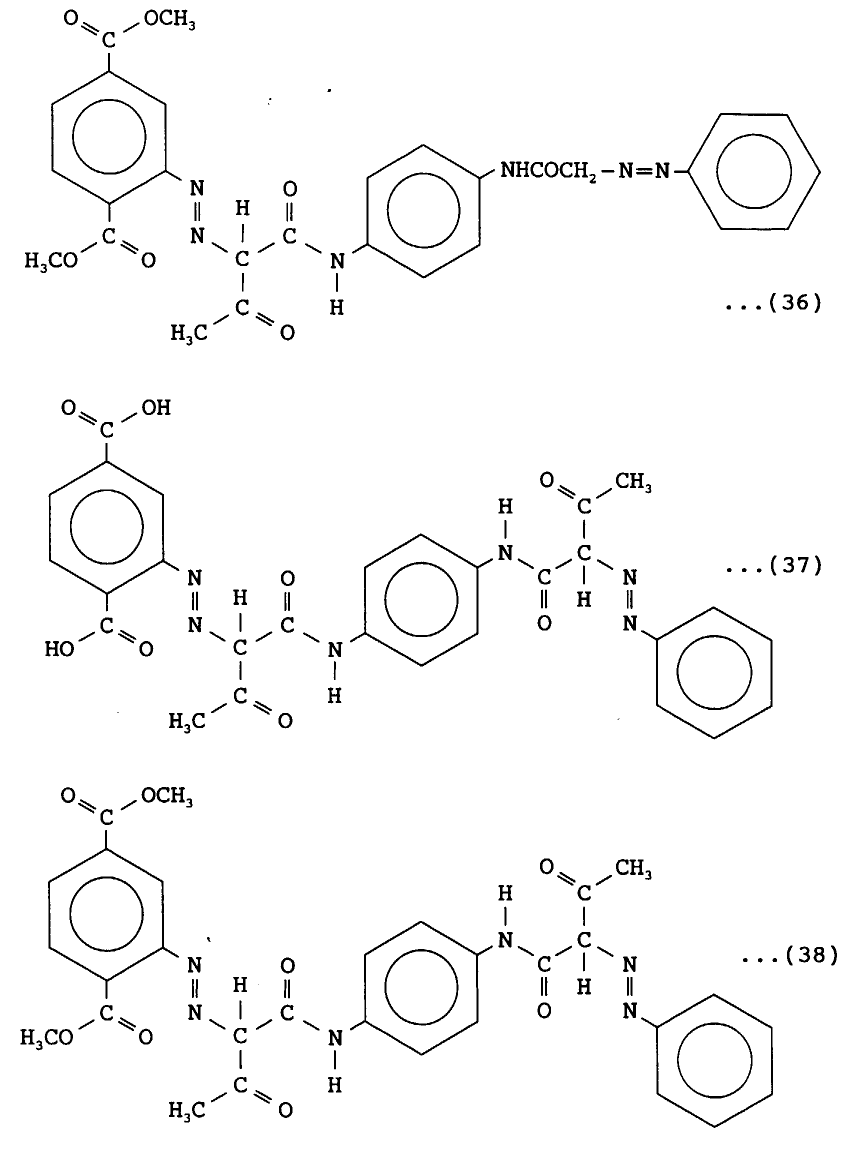 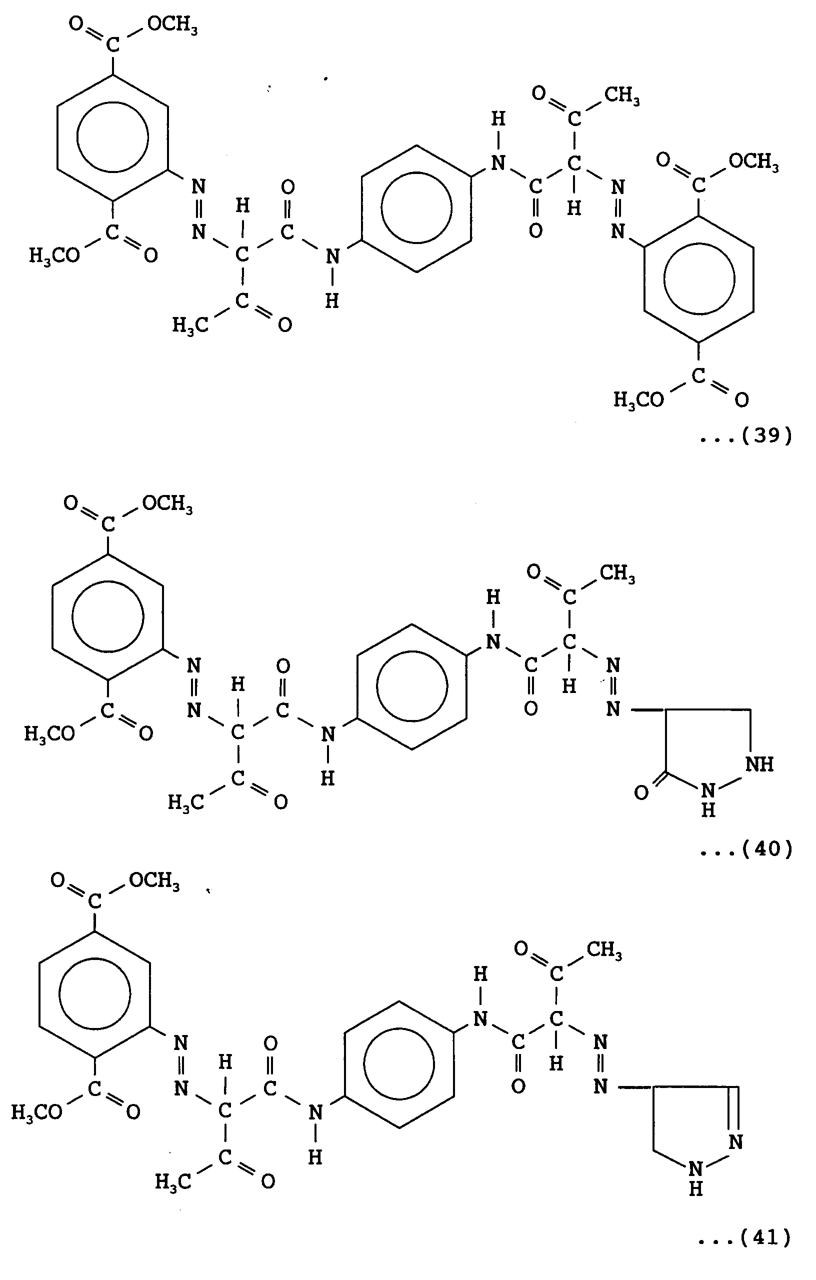 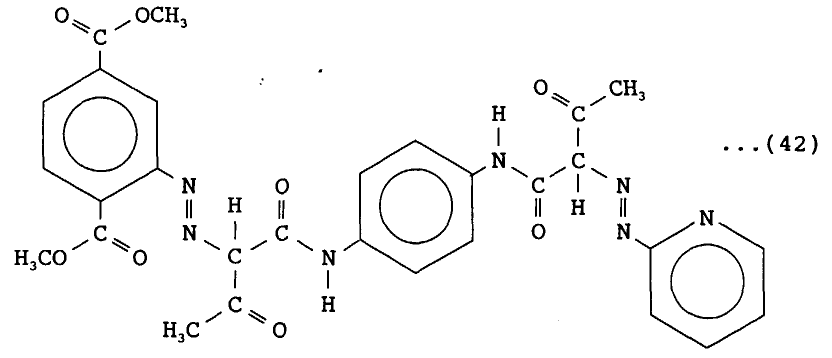 and the like.

The pigment (2) in the present invention is preferably a compound of the general formula (2) in which R³ to R⁵ are all a methyl group and n is 0, particularly preferably a compound represented by the formula (39).

The compound represented by the formula (39) corresponds to C.I. Pigment Yellow 155 according to the Color Index.

When the pigment (1) and the pigment (2) are used in combination in the present invention, the ratio of the pigment (1) to the pigment (2) is generally 95/5 to 5/95, preferably 80/20 to 20/80, particularly preferably 65/35 to 35/65.

The cyclic diketone compound as the pigment (3) is, for example, a compound having a diketopyrrolopyrrole structure, anthanthrone derivative or the like.

The above diketopyrrolopyrrole structure is represented by the following formula (43).

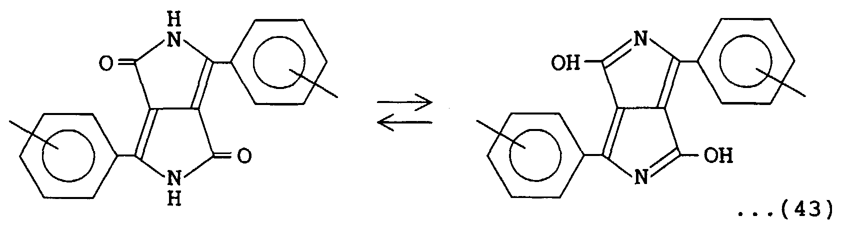

Specific examples of the pigment (3) composed of a compound having such a diketopyrrolopyrrole structure include C.I. Pigment Red 254 and the like according to Color Index.

The anthanthrone derivatives include halides of anthanthrone and the like.

Specific examples of the pigment (3) composed of a halide of anthanthrone include C.I. Pigment Red 168 represented by the following formula (44) 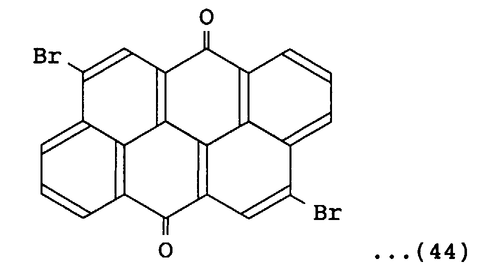 and the like.

In the above formula (3) representing the pigment (4), u and v are independently an alkyl group having 1 to 10 carbon atoms, a substituted or unsubstituted monovalent aromatic hydrocarbon group having 6 to 20 carbon atoms, or a group having a substituted or unsubstituted monovalent 5- or 6-membered nitrogen-containing heterocyclic group.

The alkyl group having 1 to 10 carbon atoms, represented by u and v, is, for example, methyl group, ethyl group, n-propyl group, i-propyl group, n-butyl group, i-butyl group, sec-butyl group, t-butyl group, n-pentyl group, n-hexyl group, n-heptyl group, n-octyl group, 2-ethylhexyl group, n-nonyl group, n-decyl group or the like.

The monovalent aromatic hydrocarbon group having 6 to 20 carbon atoms, represented by u and v, is, for example, phenyl group, 4-biphenyl group, 1-naphthyl group, 2-naphthyl group, 1-anthranyl group, 2-anthranyl group, 9-anthranyl group, 9-phenanthryl group or the like.

Substituent groups for the above aromatic hydrocarbon group include, for example, a halogen atom, nitro group, cyano group, alkyl group having 1 to 10 carbon atoms, -OR¹⁶, -COR¹⁷, -COOR¹⁸, -N(R¹⁹)(R²⁰), -SO₃R²¹, -SO₂R²², -OCOR²³, -N=NR²⁴ (in which R¹⁶ to R²¹ are independently a hydrogen atom, alkyl group having 1 to 5 carbon atoms, aryl group having 6 to 12 carbon atoms or aralkyl group having 7 to 13 carbon atoms, R²² and R²³ are independently an alkyl group having 1 to 5 carbon atoms, aryl group having 6 to 12 carbon atoms or aralkyl group having 7 to 13 carbon atoms, and R²⁴ is a monovalent aromatic hydrocarbon group having 6 to 20 carbon atoms) and the like.

Specific examples of the monovalent aromatic hydrocarbon group having 6 to 20 carbon atoms, represented by u and v, include p-chlorophenyl group, 3,5-dichlorophenyl group, p-nitrophenyl group, 3,5-dinitrophenyl group, p-cyanophenyl group, 3,5-dicyanophenyl group, o-methylphenyl group, m-methylphenyl group, p-methylphenyl group, 3,5-dimethylphenyl group, p-ethylphenyl group, 3,5-diethylphenyl group, o-hydroxyphenyl group, m-hydroxyphenyl group, p-hydroxyphenyl group, 3,5-dihydroxyphenyl group, o-methoxyphenyl group, m-methoxyphenyl group, p-methoxyphenyl group, 3,5-dimethoxyphenyl group, p-ethoxyphenyl group, 3,5-diethoxyphenyl group, p-formylphenyl group, 3,5-diformylphenyl group, p-methylcarbonylphenyl group, 3,5-dimethylcarbonylphenyl group, p-carboxyphenyl group, 3,5-dicarboxyphenyl group, p-methoxycarbonylphenyl group, 3,5-dimethoxycarbonylphenyl group, p-ethoxycarbonylphenyl group, 3,5-diethoxycarbonylphenyl group, p-aminophenyl group, 3,5-diaminophenyl group, p-dimethylaminophenyl group, 3,5-bis(dimethylamino)phenyl group, p-sulfophenyl group, 3,5-disulfophenyl group, p-methylsulfophenyl group, 3,5-dimethylsulfophenyl group, p-methylsulfonylphenyl group, 3,5-dimethylsulfonylphenyl group, p-methylcarbonyloxyphenyl group, 3,5-dimethylcarbonyloxyphenyl group, substituted phenyl group such as a group represented by the following formula (45): 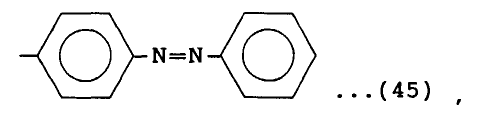 4'-chloro-4-biphenyl group, 4'-nitro-4-biphenyl group, 4'-cyano-4-biphenyl group, 4'-methyl-4-biphenyl group, 4'-hydroxy-4-biphenyl group, 4'-methoxy-4-biphenyl group, 4'-formyl-4-biphenyl group, 4'-methylcarbonyl-4-biphenyl group, 4'-carboxy-4-biphenyl group, 4'-methoxycarbonyl-4-biphenyl group, 4'-amino-4-biphenyl group, 4'-dimethylamino-4-biphenyl group, 4'-sulfo-4-biphenyl group, 4'-methylsulfo-4-biphenyl group, 4'-methylsulfonyl-4-biphenyl group, 4'-methylcarbonyloxy-4-biphenyl group, substituted 4-biphenyl group such as a group represented by the following formula (46): 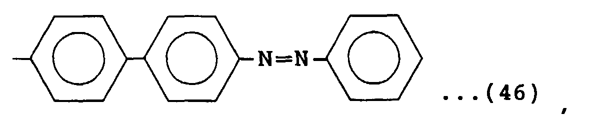 4-chloro-1-naphthyl group, 4-nitro-1-naphthyl group, 4-cyano-1-naphthyl group, 4-methyl-1-naphthyl group, 4-hydroxy-1-naphthyl group, 4-methoxy-1-naphthyl group, 4-formyl-1-naphthyl group, 4-methylcarbonyl-1-naphthyl group, 4-carboxy-1-naphthyl group, 4-methoxycarbonyl-1-naphthyl group, 4-amino-1-naphthyl group, 4-dimethylamino-1-naphthyl group, 4-sulfo-1-naphthyl group, 4-methylsulfo-1-naphthyl group, 4-methylsulfonyl-1-naphthyl group, 4-methylcarbonyloxy-1-naphthyl group, substituted 1-naphthyl group such as a group represented by the following formula (47): 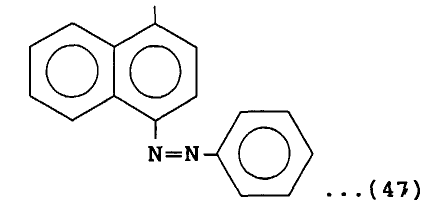 and the like.

The group having a 5- or 6-membered nitrogen-containing heterocyclic group, represented by u and v, is, for example, 1-pyrrolyl, 2-pyrrolyl, 3-pyrrolyl, 1-pyrazolyl, 3-pyrazolyl, 4-pyrazolyl, 1-imidazolyl, 2-imidazolyl, 4-imidazolyl, 1-triazolyl, 4-triazolyl, 5-triazolyl, 2-pyridinyl, 3-pyridinyl, 4-pyridinyl, 2-pyrimidinyl, 4-pyrimidinyl, 5-pyrimidinyl, 6-pyrimidinyl, 2-pyrazinyl, 3-pyrazinyl, s-triazinyl, 2-oxazolyl, 4-oxazolyl, 5-oxazolyl, 3-isooxazolyl, 4-isooxazolyl, 5-isooxazolyl, 2-thiazolyl, 4-thiazolyl, 5-thiazolyl, 3-isothiazolyl, 4-isothiazolyl, 5-isothiazolyl or the like.

Of substituent groups for the group having a nitrogen-containing heterocyclic group, substituent groups for the nitrogen atom of the above group having a nitrogen-containing heterocyclic group include, for example, an alkyl group having 1 to 5 carbon atoms, aryl group having 6 to 12 carbon atoms, aralkyl group having 7 to 13 carbon atoms and the like; and substituent groups for the carbon atom of the group having a nitrogen-containing heterocyclic group include, for example, an alkyl group having 1 to 5 carbon atoms, aryl group having 6 to 12 carbon atoms, aralkyl group having 7 to 13 carbon atoms and the same groups as substituent groups (excluding alkyl group) in the substituted derivative of the aromatic hydrocarbon group.

Specific examples of the group having a 5- or 6-membered nitrogen-containing heterocyclic group, represented by u and v, include alkyl substituted groups having a nitrogen-containing heterocyclic group, such as 2-methyl-1-pyrrolyl, 3-methyl-1-pyrrolyl, 1-methyl-2-pyrrolyl, 3-methyl-1-pyrazolyl, 4-methyl-1-pyrazolyl, 1-methyl-3-pyrazolyl, 2-methyl-1-imidazolyl, 4-methyl-1-imidazolyl, 1-methyl-2-imidazolyl, 4-methyl-2-imidazolyl, 4-methyl-1-triazolyl, 1-methyl-4-triazolyl, 4-methyl-2-pyridinyl, 2-methyl-4-pyridinyl, 4-methyl-2-pyrimidinyl, 2-methyl-4-pyrimidinyl, 3-methyl-2-pyrazinyl, 2-methyl-3-pyrazinyl, methyl-s-triazinyl and dimethyl-s-triazinyl; phenyl substituted groups having a nitrogen-containing heterocyclic group, such as 2-phenyl-1-pyrrolyl, 3-phenyl-1-pyrrolyl, 1-phenyl-2-pyrrolyl, 3-phenyl-1-pyrazolyl, 4-phenyl-1-pyrazolyl, 1-phenyl-3-pyrazolyl, 2-phenyl-1-imidazolyl, 4-phenyl-1-imidazolyl, 1-phenyl-2-imidazolyl, 4-phenyl-2-imidazolyl, 4-phenyl-1-triazolyl, 1-phenyl-4-triazolyl, 4-phenyl-2-pyridinyl, 2-phenyl-4-pyridinyl, 4-phenyl-2-pyrimidinyl, 2-phenyl-4-pyrimidinyl, 3-phenyl-2-pyrazinyl, 2-phenyl-3-pyrazinyl, phenyl-s-triazinyl and diphenyl-s-triazinyl; benzyl substituted groups having a nitrogen-containing heterocyclic group, such as 2-benzyl-1-pyrrolyl, 3-benzyl-1-pyrrolyl, 1-benzyl-2-pyrrolyl, 3-benzyl-1-pyrazolyl, 4-benzyl-1-pyrazolyl, 1-benzyl-3-pyrazolyl, 2-benzyl-1-imidazolyl, 4-benzyl-1-imidazolyl, 1-benzyl-2-imidazolyl, 4-benzyl-2-imidazolyl, 4-benzyl-1-triazolyl, 1-benzyl-4-triazolyl, 4-benzyl-2-pyridinyl, 2-benzyl-4-pyridinyl, 4-benzyl-2-pyrimidinyl, 2-benzyl-4-pyrimidinyl, 3-benzyl-2-pyrazinyl, 2-benzyl-3-pyrazinyl, benzyl-s-triazinyl and dibenzyl-s-triazinyl; and the like. U and v in the above formula (3) are preferably methyl group, ethyl group, o-methylphenyl group, m-methylphenyl group, p-methylphenyl group, 3,5-dimethylphenyl group, o-methoxyphenyl group, m-methoxyphenyl group, p-methoxyphenyl group, 3,5-dimethoxylphenyl group, p-ethoxyphenyl group, 3,5-diethoxylphenyl group or the group represented by the above formula (45), and more preferably methyl group, 3,5-dimethylphenyl group, p-ethoxyphenyl group or the group represented by the above formula (45).

Specific examples of the pigment (4) include C.I. Pigment Red 123 (u and v are both p-ethoxyphenyl group), C.I. Pigment Red 149 (u and v are both 3,5-dimethylphenyl group), C.I. Pigment Red 178 (u and v are both the group represented by the formula (45)), C.I. Pigment Red 179 (u and v are both methyl group) and the like according to the Color Index.

The perylene pigment in the present invention is particularly preferably a pigment (5) represented by the above formula (4). The pigment (5) corresponds to C.I. Pigment Red 224.

In the present invention, the above perylene pigments can be used alone or in admixture of two or more.

In the present invention, other colorant can be used in combination with the pigment (1), (2), (3), (4) or (5), as the case may be.

The other colorant is not limited to a particular color and may be either organic or inorganic colorant.

The organic colorant is a dye, organic pigment, natural coloring matter or the like. The inorganic colorant is an inorganic pigment, inorganic salt which is called an extender pigment, or the like. The other colorant in the present invention is preferably a colorant having high color developability and heat resistance, particularly preferably a colorant having high resistant to heat decomposition. The colorant is generally an organic colorant, particularly preferably an organic pigment.

Specific examples of the organic pigment include pigments of the following Color Index numbers.

C.I. Pigment Yellow 1, C.I. Pigment Yellow 3, C.I. Pigment Yellow 12, C.I. Pigment Yellow 13, C.I. Pigment Yellow 14, C.I. Pigment Yellow 16, C.I. Pigment Yellow 17, C.I. Pigment Yellow 20, C.I. Pigment Yellow 24, C.I. Pigment Yellow 31, C.I. Pigment Yellow 55, C.I. Pigment Yellow 60, C.I. Pigment Yellow 65, C.I. Pigment Yellow 73, C.I. Pigment Yellow 74, C.I. Pigment Yellow 81, C.I. Pigment Yellow 83, C.I. Pigment Yellow 93, C.I. Pigment Yellow 95, C.I. Pigment Yellow 97, C.I. Pigment Yellow 98, C.I. Pigment Yellow 100, C.I. Pigment Yellow 101, C.I. Pigment Yellow 104, C.I. Pigment Yellow 106, C.I. Pigment Yellow 108, C.I. Pigment Yellow 109, C.I. Pigment Yellow 110, C.I. Pigment Yellow 113, C.I. Pigment Yellow 114, C.I. Pigment Yellow 116, C.I. Pigment Yellow 117, C.I. Pigment Yellow 119, C.I. Pigment Yellow 120, C.I. Pigment Yellow 126, C.I. Pigment Yellow 127, C.I. Pigment Yellow 128, C.I. Pigment Yellow 129, C.I. Pigment Yellow 138, C.I. Pigment Yellow 139, C.I. Pigment Yellow 150, C.I. Pigment Yellow 151, C.I. Pigment Yellow 152, C.I. Pigment Yellow 153, C.I. Pigment Yellow 154, C.I. Pigment Yellow 155, C.I. Pigment Yellow 156, C.I. Pigment Yellow 166, C.I. Pigment Yellow 168, and C.I. Pigment Yellow 175; C.I. Pigment Orange 36, C.I. Pigment Orange 43, C.I. Pigment Orange 51, C.I. Pigment Orange 61, C.I. Pigment Orange 71 and C.I. Pigment Orange 73; C.I. Pigment Red 9, C.I. Pigment Red 97, C.I. Pigment Red 122, C.I. Pigment Red 123, C.I. Pigment Red 149, C.I. Pigment Red 168, C.I. Pigment Red 176, C.I. Pigment Red 177, C.I. Pigment Red 180, C.I. Pigment Red 202, C.I. Pigment Red 209, C.I. Pigment Red 215, C.I. Pigment Red 242, C.I. Pigment Red 254, C.I. Pigment Red 255 and C.I. Pigment Red 265; C.I. Pigment Violet 19, C.I. Pigment Violet 23 and C.I. Pigment Violet 29; C.I. Pigment Blue 15 and C.I. Pigment Blue 15:3, C.I. Pigment Blue 15:4, C.I. Pigment Blue 15:6, C.I. Pigment Blue 60; C.I. Pigment Green 7 and C.I. Pigment Green 36; C.I. Pigment Brown 23 and C.I. Pigment Brown 25; and C.I. Pigment Black 1 and C.I. Pigment Black 7.

Specific examples of the inorganic colorant include titanium oxide, barium sulfate, calcium carbonate, zinc white, lead sulfate, yellow lead, zinc yellow, red oxide (red iron oxide (III)), cadmium red, ultramarine blue, iron blue, chromium oxide green, cobalt green, amber, titanium black, synthetic iron black, carbon black and the like.

In the present invention, these colorants can be used alone or in admixture of two or more.

The amount of the other colorant used is generally 99 wt% or less, preferably 90 wt% or less, based on the total amount of the colorants. When the amount of the other colorant is more than 99 wt%, it is difficult to ensure satisfactory coatability, sensitivity, developability and the like while well-balanced chroma and lightness being kept.

In the present invention, the colorant can be used in combination with a dispersant as desired. The dispersant is, for example, a cationic, anionic, nonionic, amphoteric, silicone-based or fluorine-based surfactant.

Specific examples of the surfactant include polyoxyethylene alkyl ethers such as polyoxyethylene lauryl ether, polyoxyethylene stearyl ether and polyoxyethylene oleyl ether; polyoxyethylene alkylphenyl ethers such as polyoxyethylene octylphenyl ether and polyxoyethylene nonylphenyl ether; polyethylene glycol diesters such as polyethylene glycol dilaurate and polyethylene glycol distearate; sorbitan fatty acid esters; fatty acid-modified polyesters; tertiary amine modified polyurethanes; polyethylene imines; and the like. There can be also usable those available under the trade name of KP (a product of Shin-Etsu Chemical Co., Ltd.), Polyflow (a product of Kyoei Yushi Kagaku Kogyo K.K.), F-Top (a product of Tochem Products Co., Ltd.), Megafac (a product of Dainippon Ink and Chemicals, Inc.), Florade (a product of Sumitomo 3M Limited), Asahi Guard and Surflon (products of Asahi Glass Co., Ltd.) and the like.

These surfactants can be used alone or in admixture of two or more.

The amount of the surfactant used is generally 50 parts or less by weight, preferably 0 to 40 parts by weight, based on 100 parts by weight of the colorant.

### (B) Alkali-soluble resin

As the alkali-soluble resin in the present invention, any resin can be used so long as it serves as a binder for the colorant (A) and is soluble in an alkali developer used in a development process in the production of a color filter.

The alkali-soluble resin is, for example, a resin having an acidic functional group such as a carboxyl group or phenolic hydroxyl group.

Of alkali-soluble resins, alkali-soluble resins having a carboxyl group include, for example, (co)polymers of ethylenically unsaturated monomers having more than one carboxyl group (to be simply referred to as "carboxyl group-containing unsaturated monomers" hereinafter).

As the (co)polymers of the carboxyl group-containing unsaturated monomers, particularly preferred are a copolymer (to be simply referred to as "carboxyl group-containing copolymer (b)" hereinafter) of a monomer mixture of a carboxyl group-containing unsaturated monomer and other copolymerizable ethylenically unsaturated monomer (to be simply referred to as "other unsaturated monomer" hereinafter).

Specific examples of the above carboxyl group-containing unsaturated monomer include unsaturated monocarboxylic acids such as acrylic acid, methacrylic acid, crotonic acid, α-chloroacrylic acid, ethacrylic acid and cinammic acid; unsaturated dicarboxylic acids (anhydrides) such as maleic acid, maleic anhydride, fumaric acid, itaconic acid, itaconic anhydride, citraconic acid, citraconic anhydride and mesaconic acid; unsaturated polycarboxylic acids (anhydrides) having at least three carboxyl groups; mono[(meth)acryloyloxyalkyl]esters of polycarboxylic acids having at least two carboxyl groups, such as mono(2-acryloyloxyethyl) succinate, mono(2-methacryloyloxyethyl) succinate, mono(2-acryloyloxyethyl) phathalate and mono(2-methacryloyloxyethyl) phthalate; mono(meth)acrylates of polymers having carboxyl groups at both terminals such as ω-carboxy-polycaprolactone monoacrylate and ω-carboxy-polycaprolactone monomethacrylate; and the like.

These carboxyl group-containing unsaturated monomers can be used alone or in admixture of two or more.

Specific examples of the other unsaturated monomer include aromatic vinyl compounds such as styrene, α-methylstyrene, o-vinyltoluene, m-vinyltoluene, p-vinyltoluene, o-chlorostyrene, m-chlorostyrene, p-chlorostyrene, o-methoxystyrene, m-methoxystyrene, p-methoxystyrene, indene, o-vinylbenzylmethyl ether, m-vinylbenzylmethyl ether, p-vinylbenzylmethyl ether, o-vinylbenzylglycidyl ether, m-vinylbenzylglycidyl ether, p-vinylbenzylglycidyl ether or the like; unsaturated carboxylic acid esters such as methyl acrylate, methyl methacrylate, ethyl acrylate, ethyl methacrylate, n-propyl acrylate, n-propyl methacrylate, i-propyl acrylate, i-propyl methacrylate, n-butyl acrylate, n-butyl methacrylate, i-butyl acrylate, i-butyl methacrylate, sec-butyl acrylate, sec-butyl methacrylate, t-butyl acrylate, t-butyl methacrylate, 2-hydroxyethyl acrylate, 2-hydroxyethyl methacrylate, 2-hydroxypropyl acrylate, 2-hydroxypropyl methacrylate, 3-hydroxypropyl acrylate, 3-hydroxypropyl methacrylate, 2-hydroxybutyl acrylate, 2-hydroxybutyl methacrylate, 3-hydroxybutyl acrylate, 3-hydroxybutyl methacrylate, 4-hydroxybutyl acrylate, 4-hydroxybutyl methacrylate, allyl acrylate, allyl methacrylate, benzyl acrylate, benzyl methacrylate, cyclohexyl acrylate, cyclohexyl methacrylate, phenyl acrylate, phenyl methacrylate, 2-methoxyethyl acrylate, 2-methoxyethyl methacrylate, 2-phenoxyethyl acrylate, 2-phenoxyethyl methacrylate, methoxydiethylene glycol acrylate, methoxydiethylene glycol methacrylate, methoxytriethylene glycol acrylate, methoxytriethylene glycol methacrylate, methoxypropylene glycol acrylate, methoxypropylene glycol methacrylate, methoxydipropylene glycol acrylate, methoxydipropylene glycol methacrylate, isobornyl acrylate, isobornyl methacrylate, dicyclopentadienyl acrylate, dicyclopentadienyl methacrylate, 2-hydroxy-3-phenoxypropyl acrylate, 2-hydroxy-3-phenoxypropyl methacrylate or the like; unsaturated carboxylic acid aminoalkyl esters such as 2-aminoethyl acrylate, 2-aminoethyl methacrylate, 2-dimethylaminoethyl acrylate, 2-dimethylaminoethyl methacrylate, 2-aminopropyl acrylate, 2-aminopropyl methacrylate, 2-dimethylaminopropyl acrylate, 2-dimethylaminopropyl methacrylate, 3-aminopropyl acrylate, 3-aminopropyl methacrylate, 3-dimethylaminopropyl acrylate, 3-dimethylaminopropyl methacrylate or the like; unsaturated carboxylic acid glycidyl esters such as glycidyl acrylate, glycidyl methacrylate or the like; carboxylic acid vinyl esters such as vinyl acetate, vinyl propionate, vinyl butyrate, vinyl benzoate or the like; unsaturated ethers such as vinylmethyl ether, vinylethyl ether, allylglycidyl ether, methallylglycidyl ether or the like; vinyl cyanide compounds such as acrylonitrile, methacrylonitrile, α-chloroacrylonitrile, vinylidene cyanide or the like; unsaturated amides such as acrylamide, methacrylamide, α-chloroacrylamide, N-(2-hydroxyethyl)acrylamide, N-(2-hydroxyethyl)methacrylamide or the like; unsaturated imides such as maleimide, N-cyclohexyl maleimide, N-phenyl maleimide or the like; aliphatic conjugated dienes such as 1,3-butadiene, isoprene, chloroprene or the like; macromonomers having a monoacryloyl group or monomethacryloyl group at the terminal(s) such as polystyrene, polymethyl acrylate, polymethyl methacrylate, poly-n-butyl acrylate, poly-n-butyl methacrylate, polysilicone or the like; and the like.

These other unsaturated monomers can be used alone or in admixture of two or more.

Preferably, the carboxyl group-containing copolymer (b) is a copolymer of (1) an acrylic acid and/or methacrylic acid and (2) at least one member selected from the group consisting of styrene, methyl acrylate, methyl methacrylate, 2-hydroxyethyl acrylate, 2-hydroxyethyl methacrylate, benzyl acrylate, benzyl methacrylate, phenyl acrylate, phenyl methacrylate, N-phenyl maleimide, polystyrene macromonomer and polymethyl methacrylate macromonomer.

Preferred examples of the carboxyl group-containing copolymer (b) include acrylic acid copolymers such as an acrylic acid/benzyl acrylate copolymer, acrylic acid/styrene/methyl acrylate copolymer, acrylic acid/styrene/benzyl acrylate copolymer, acrylic acid/methyl acrylate/polystyrene macromonomer copolymer, acrylic acid/methyl acrylate/polymethyl methacrylate macromonomer copolymer, acrylic acid/benzyl acrylate/polystyrene macromonomer copolymer, acrylic acid/benzyl acrylate/polymethyl methacrylate macromonomer copolymer, acrylic acid/benzyl methacrylate copolymer, acrylic acid/styrene/methyl methacrylate copolymer, acrylic acid/styrene/benzyl methacrylate copolymer, acrylic acid/methyl methacrylate/polystyrene macromonomer copolymer, acrylic acid/methyl methacrylate/polymethyl methacrylate macromonomer copolymer, acrylic acid/benzyl methacrylate/polystyrene macromonomer copolymer, acrylic acid/benzyl methacrylate/polymethyl methacrylate macromonomer copolymer, acrylic acid/2-hydroxyethyl methacrylate/benzyl methacrylate/polystyrene macromonomer copolymer, acrylic acid/2-hydroxyethyl methacrylate/benzyl methacrylate/polymethyl methacrylate macromonomer copolymer or the like; and methacrylic acid copolymers such as methacrylic acid/benzyl acrylate copolymer, methacrylic acid/styrene/methyl acrylate copolymer, methacrylic acid/styrene/benzyl acrylate copolymer, methacrylic acid/methyl acrylate/polystyrene macromonomer copolymer, methacrylic acid/methyl acrylate/polymethyl methacrylate macromonomer copolymer, methacrylic acid/benzyl acrylate/polystyrene macromonomer copolymer, methacrylate acid/benzyl acrylate/polymethyl methacrylate macromonomer copolymer, methacrylic acid/benzyl methacrylate copolymer, methacrylic acid/styrene/methyl methacrylate copolymer, methacrylic acid/styrene/benzyl methacrylate copolymer, methacrylic acid/methyl methacrylate/polystyrene macromonomer copolymer, methacrylic acid/methyl methacrylate/polymethyl methacrylate macromonomer copolymer, methacrylic acid/benzyl methacrylate/polystyrene macromonomer copolymer, methacrylic acid/benzyl methacrylate/polymethyl methacrylate macromonomer copolymer, methacrylic acid/2-hydroxyethyl methacrylate/benzyl methacrylate/polystyrene macromonomer copolymer, methacrylic acid/2-hydroxyethyl methacrylate/benzyl methacrylate/polymethyl methacrylate macromonomer copolymer, methacrylic acid/styrene/benzyl methacrylate/N-phenyl maleimide copolymer, methacrylic acid/styrene/phenyl methacrylate/N-phenyl maleimide copolymer, methacrylic acid/styrene/benzyl methacrylate/N-phenyl maleimide/polystyrene macromonomer copolymer, methacrylic acid/styrene/benzyl methacrylate/N-phenyl maleimide/polymethyl methacrylate macromonomer copolymer, methacrylic acid/styrene/phenyl methacrylate/N-phenyl maleimide/polystyrene macromonomer copolymer, methacrylic acid/styrene/phenyl methacrylate/N-phenyl maleimide/polymethyl methacrylate macromonomer copolymer, methacrylic acid/styrene/2-hydroxyethyl methacrylate/benzyl methacrylate/N-phenyl maleimide/polystyrene macromonomer copolymer, methacrylic acid/styrene/2-hydroxyethyl methacrylate/benzyl methacrylate/N-phenyl maleimide/polymethyl methacrylate macromonomer copolymer, methacrylic acid/styrene/2-hydroxyethyl methacrylate/phenyl methacrylate/N-phenyl maleimide/polystyrene macromonomer copolymer, methacrylic acid/styrene/2-hydroxyethyl methacrylate/phenyl methacrylate/N-phenyl maleimide/polymethyl methacrylate macromonomer copolymer or the like.

Of these carboxyl group-containing copolymers (b), particularly preferred are methacrylic acid/benzyl methacrylate copolymer, methacrylic acid/styrene/methyl methacrylate copolymer, methacrylic acid/styrene/benzyl methacrylate copolymer, methacrylic acid/methyl methacrylate/polystyrene macromonomer copolymer, methacrylic acid/methyl methacrylate/polymethyl methacrylate macromonomer copolymer, methacrylic acid/benzyl methacrylate/polystyrene macromonomer copolymer, methacrylic acid/benzyl methacrylate/polymethyl methacrylate macromonomer copolymer, methacrylic acid/2-hydroxyethyl methacrylate/benzyl methacrylate/polystyrene macromonomer copolymer, methacrylic acid/2-hydroxyethyl methacrylate/benzyl methacrylate/polymethyl methacrylate macromonomer copolymer, methacrylic acid/styrene/benzyl methacrylate/N-phenyl maleimide copolymer, methacrylic acid/styrene/phenyl methacrylate/N-phenyl maleimide copolymer, methacrylic acid/styrene/benzyl methacrylate/N-phenyl maleimide/polystyrene macromonomer copolymer, methacrylic acid/styrene/benzyl methacrylate/N-phenyl maleimide/polymethyl methacrylate macromonomer copolymer, methacrylic acid/styrene/phenyl methacrylate/N-phenyl maleimide/polystyrene macromonomer copolymer, methacrylic acid/styrene/phenyl methacrylate/N-phenyl maleimide/polymethyl methacrylate macromonomer copolymer, methacrylic acid/styrene/2-hydroxyethyl methacrylate/benzyl methacrylate/N-phenyl maleimide/polystyrene macromonomer copolymer, methacrylic acid/styrene/2-hydroxyethyl methacrylate/benzyl methacrylate/N-phenyl maleimide/polymethyl methacrylate macromonomer copolymer, methacrylic acid/styrene/2-hydroxyethyl methacrylate/phenyl methacrylate/N-phenyl maleimide/polystyrene macromonomer copolymer, and methacrylic acid/styrene/2-hydroxyethyl methacrylate/phenyl methacrylate/N-phenyl maleimide/polymethyl methacrylate macromonomer copolymer.

The amount of the carboxyl group-containing unsaturated monomer is generally 5 to 50 wt%, preferably 10 to 40 wt% of the carboxyl group-containing copolymer (b). When the amount of the carboxyl group-containing unsaturated monomer is less than 5 wt%, the solubility in an alkali developer of the obtained radiation sensitive composition is liable to decline, while when the amount is more than 50 wt%, the formed pixels are liable to fall off from the substrate, or the surface of each of the pixels is liable to be roughened at the time of development with an alkali developer.

Particularly, the carboxyl group-containing copolymer (b) containing the carboxyl group-containing unsaturated monomer in the above specified proportion has excellent solubility in an alkali developer. In the radiation sensitive composition comprising the above copolymer as a binder, undissolved substance rarely remains after development with an alkali developer, surface soiling or resin remaining are hardly produced in an area other than portion(s) where pixels are formed, of the substrate, and pixels obtained from the composition do not dissolve excessively in an alkali developer and have excellent adhesion to the substrate. As a result, the pixels do not fall off from the substrate.

The carboxyl group-containing copolymer (b) has a weight-average molecular weight calculated as polystyrene (to be simply referred to as "weight-average molecular weight" hereinafter), measured by a gel permeation chromatography (GPC)(elution solvent: tetrahydrofuran), of preferably 3,000 to 300,000, more preferably 5,000 to 100,000.

An alkali-soluble polyester resin can be used as the alkali-soluble resin.

Such polyester resin is particularly preferable when it is a polylactic acid having a relatively low molecular weight.

The weight-average molecular weight Of the polylactic acid is generally 3,000 to 300,000, preferably 5,000 to 100,000.

Phenolic hydroxyl group-containing alkali-soluble resins include, for example, (co)polymers of phenolic hydroxyl group-containing vinyl aromatic compounds, phenolic novolak resins and the like.

The phenolic hydroxyl group-containing vinyl aromatic compounds include, for example, o-hydroxystyrene, m-hydroxystyrene, p-hydroxystyrene, o-hydroxy-α-methylstyrene, m-hydroxy-α-methylstyrene, p-hydroxyl-α-methylstyrene and the like.

These phenolic hydroxyl group-containing vinyl aromatic compounds can be used alone or in admixture of two or more.

The phenolic hydroxyl group-containing vinyl aromatic compounds can be copolymerized with at least one other copolymerizable ethylenically unsaturated monomer, for example, the other unsaturated monomers listed for the carboxyl group-containing copolymer (b), as required.

Phenols used in the phenolic novolak resins include, for example, o-cresol, m-cresol, p-cresol, 2,3-xylenol, 2,4-xylenol, 2,5-xylenol, 3,4-xylenol, 3,5-xylenol, 2,3,5-trimethylphenol, 3,4,5-trimethylphenol and the like and aldehydes used in the phenolic novolak resins include formaldehyde, trioxane, paraformaldehyde, benzaldehyde, acetoaldehyde, propylaldehyde, phenylacetoaldehyde, glyoxal, glutaraldehyde, terephthalaldehyde, isophthalaldehyde and the like.

These phenols and aldehydes can be used alone or in admixture of two or more.

The (co)polymers of the phenolic hydroxyl group-containing vinyl aromatic compounds preferably have a weight-average molecular weight of 1,000 to 150,000, more preferably 3,000 to 100,000.

The phenolic novolak resins preferably have a weight-average molecular weight of 1,000 to 150,000, more preferably 1,500 to 80,000.

In the present invention, the alkali-soluble resins can be used alone or in admixture of two or more.

The amount of the alkali-soluble resin used in the present invention is generally 10 to 1,000 parts by weight, preferably 20 to 500 parts by weight based on 100 parts by weight of the colorant (A). When the amount of the alkali-soluble resin is less than 10 parts by weight, alkali developability may deteriorate, and surface soiling or resin remaining may be produced in an area other than portion(s) where pixels are formed, of the substrate. When the amount is more than 1,000 parts by weight, it may be difficult to obtain a target color density of a thin film due to a relative decline in the concentration of the colorant.

### (C) Polyfunctional monomer

The polyfunctional monomer in the present invention is a monomer having two or more polymerizable, ethylenically unsaturated bonds.

Specific examples of the polyfunctional monomer include diacrylates and dimethacrylates of alkylene glycol such as ethylene glycol, propylene glycol or the like; diacrylates and dimethacrylates of polyalkylene glycol such as polyethylene glycol, polypropylene glycol or the like; polyacrylates and polymethacrylates of a polyhydric alcohol having at least three hydroxyl groups, such as glycerine, trimethylolpropane, pentaerythritol, dipentaerythritol or the like; oligoacrylates and oligomethacrylates such as polyesters, epoxy resins, urethane resins, alkyd resins, silicone resins, spiran resins or the like; diacrylates and dimethacrylates of both terminal hydroxylated polymers such as poly-1,3-butadiene having hydroxyl groups at both terminals, polyisoprene having hydroxyl groups at both terminals, polycaprolactone having hydroxyl groups at both terminals or the like; tris(2-acryloyloxyethyl)phosphate, tris(2-methacryloyloxyethyl)phosphate; and the like.

Of these polyfunctional monomers, preferred are polyacrylates and polymethacrylates of a polyhydric alcohol having at least three hydroxyl groups, such as trimethylolpropane triacrylate, trimethylolpropane trimethacrylate, pentaerythritol triacrylate, pentaerythritol trimethacrylate, pentaerythritol tetraacrylate, pentaerythritol tetramethacrylate, dipentaerythritol pentaacrylate, dipentaerythritol pentamethacrylate, dipentaerythritol hexaacrylate, dipentaerythritol hexamethacrylate and the like. Trimethylolpropane triacrylate, pentaerythritol triacrylate, dipentaerythritol pentaacrylate and dipentaerythritol hexaacrylate are particularly preferred because a high pixel strength and an excellent smooth pixel surface can be obtained and surface soiling or resin remaining is rarely produced in an area other than portion(s) where pixels are formed.

In the present invention, the polyfunctional monomers can be used alone or in admixture of two or more.

The amount of the polyfunctional monomer used in the present invention is generally 5 to 500 parts by weight, preferably 20 to 300 parts by weight, based on 100 parts by weight of the alkali-soluble resin (B). When the amount of the polyfunctional monomer is less than 5 parts by weight, pixel strength or pixel surface smoothness may be insufficient, while when the amount is more than 500 parts by weight, alkali developability may deteriorate, and surface soiling or resin remaining may be produced in an area other than portion(s) where pixels are formed, of the substrate.

### (D) Photopolymerization initiator

The photopolymerization initiator in the present invention is a compound which can generate an active species capable of starting the polymerization of the above polyfunctional monomer (C) when exposed to radiation such as visible light, ultraviolet light, far ultraviolet light, electron beam or X-ray.

Specific examples of the photopolymerization initiator include biimidazole-based compounds having at least one main skeleton represented by the following formulas (48), (49) and (50): 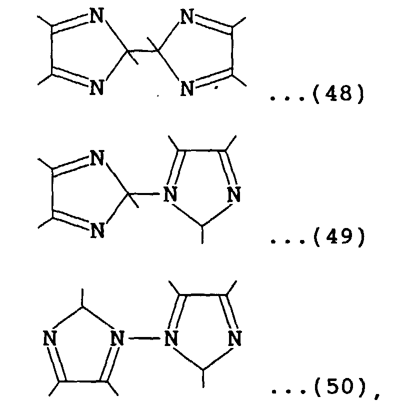 benzoin-based compounds, acetophenone-based compounds, benzophenone-based compounds, α-diketone-based compounds, polynuclear quinone-based compounds, xanthone-based compounds, diazo-based compounds, triazine-based compounds and the like. Of these, biimidazole-based compounds are preferred.

The biimidazole-based compounds include, for example, 2,2'-bis(2-chlorophenyl)-4,4',5,5'-tetrakis(4-ethoxycarbonylphenyl)-1,2'-biimidazole, 2,2'-bis(2-chlorophenyl)-4,4',5,5'-tetrakis(4-phenoxycarbonylphenyl)-1,2'-biimidazole, 2,2'-bis(2,4-dichlorophenyl)-4,4',5,5'-tetrakis(4-ethoxycarbonylphenyl)-1,2'-biimidazole, 2,2'-bis(2,4-dichlorophenyl)-4,4',5,5'-tetrakis(4-phenoxycarbonylphenyl)-1,2'-biimidazole, 2,2'-bis(2,4,6-trichlorophenyl)-4,4',5,5'-tetrakis(4-ethoxycarbonylphenyl)-1,2'-biimidazole, 2,2'-bis(2,4,6-trichlorophenyl)-4,4',5,5'-tetrakis(4-phenoxycarbonylphenyl)-1,2'-biimidazole, 2,2'-bis(2-bromophenyl)-4,4',5,5'-tetrakis(4-ethoxycarbonylphenyl)-1,2'-biimidazole, 2,2'-bis(2-bromophenyl)-4,4',5,5'-tetrakis(4-phenoxycarbonylphenyl)-1,2'-biimidazole, 2,2'-bis(2,4-dibromophenyl)-4,4',5,5'-tetrakis(4-ethoxycarbonylphenyl)-1,2'-biimidazole, 2,2'-bis(2,4-dibromophenyl)-4,4',5,5'-tetrakis(4- phenoxycarbonylphenyl)-1,2'-biimidazole, 2,2'-bis(2,4,6-tribromophenyl)-4,4',5,5'-tetrakis(4-ethoxycarbonylphenyl)-1,2'-biimidazole, 2,2'-bis(2,4,6-tribromophenyl)-4,4',5,5'-tetrakis(4-phenoxycarbonylphenyl)-1,2'-biimidazole, 2,2'-bis(2-cyanophenyl)-4,4',5,5'-tetrakis(4-ethoxycarbonylphenyl)-1,2'-biimidazole, 2,2'-bis(2-cyanophenyl)-4,4',5,5'-tetrakis(4-phenoxycarbonylphenyl)-1,2'-biimidazole, 2,2'-bis(2-methylphenyl)-4,4',5,5'-tetrakis(4-methoxycarbonylphenyl)-1,2'-biimidazole, 2,2'-bis(2-methylphenyl)-4,4',5,5'-tetrakis(4-ethoxycarbonylphenyl)-1,2'-biimidazole, 2,2'-bis(2-methylphenyl)-4,4',5,5'-tetrakis(4-phenoxycarbonylphenyl)-1,2'-biimidazole, 2,2'-bis(2-ethylphenyl)-4,4',5,5'-tetrakis(4-methoxycarbonylphenyl)-1,2'-biimidazole, 2,2'-bis(2-ethylphenyl)-4,4',5,5'-tetrakis(4-ethoxycarbonylphenyl)-1,2'-biimidazole, 2,2'-bis(2-ethylphenyl)-4,4',5,5'-tetrakis(4-phenoxycarbonylphenyl)-1,2'-biimidazole, 2,2'-bis(2-phenylphenyl)-4,4',5,5'-tetrakis(4-methoxycarbonylphenyl)-1,2'-biimidazole, 2,2'-bis(2-phenylphenyl)-4,4',5,5'-tetrakis(4-ethoxycarbonylphenyl)-1,2'-biimidazole, 2,2'-bis(2-phenylphenyl)-4,4',5,5'-tetrakis(4-phenoxycarbonylphenyl)-1,2'-biimidazole, 2,2'-bis(2,4-dichlorophenyl)-4,4',5,5'-tetraphenyl-1,2'-biimidazole, 2,2'-bis(2,4,6-trichlorophenyl)-4,4',5,5'-tetraphenyl-1,2'-biimidazole, 2,2'-bis(2,4-dibromophenyl)-4,4',5,5'-tetraphenyl-1,2'-biimidazole, 2,2'-bis(2,4,6-tribromophenyl)-4,4',5,5'-tetraphenyl-1,2'-biimidazole, 2,2'-bis(2,4-dicyanophenyl)-4,4',5,5'-tetraphenyl-1,2'-biimidazole, 2,2'-bis(2,4,6-tricyanophenyl)-4,4',5,5'-tetraphenyl-1,2'-biimidazole, 2,2'-bis(2,4-dimethylphenyl)-4,4',5,5'-tetraphenyl-1,2'-biimidazole, 2,2'-bis(2,4,6-trimethylphenyl)-4,4',5,5'-tetraphenyl-1,2'-biimidazole, 2,2'-bis(2,4-diethylphenyl)-4,4',5,5'-tetraphenyl-1,2'-biimidazole, 2,2'-bis(2,4,6-triethylphenyl)-4,4',5,5'-tetraphenyl-1,2'-biimidazole, 2,2'-bis(2,4-diphenylphenyl)-4,4',5,5'-tetraphenyl-1,2'-biimidazole, 2,2'-bis(2,4,6-triphenylphenyl)-4,4',5,5'-tetraphenyl-1,2'-biimidazole, and the like.

Of these biimidazole-based compounds, particularly preferred are 2,2'-bis(2-chlorophenyl)-4,4',5,5'-tetrakis(4-ethoxycarbonylphenyl)-1,2'-biimidazole, 2,2'-bis(2-bromophenyl)-4,4',5,5'-tetrakis(4-ethoxycarbonylphenyl)-1,2'-biimidazole, 2,2'-bis(2,4-dichlorophenyl)-4,4',5,5'-tetraphenyl-1,2'-biimidazole, 2,2'-bis(2,4,6-trichlorophenyl)-4,4',5,5'-tetraphenyl-1,2'-biimidazole, 2,2'-bis(2,4-dibromophenyl)-4,4',5,5'-tetraphenyl-1,2'-biimidazole and 2,2'-bis(2,4,6-tribromophenyl)-4,4',5,5'-tetraphenyl-1,2'-biimidazole.

The biimidazole-based compounds have excellent solubility in a solvent, do not produce foreign matters such as undissolved portions or precipitates, are very sensitive, allow a curing reaction to proceed sufficiently by exposure with a small amount of energy, give a high contract and do not cause a curing reaction in unexposed portions. Therefore, after exposure, the coating film is clearly divided into cured portions insoluble in a developer and uncured portions having high solubility in the developer, whereby a color filter free from a partial or complete loss of a pattern and having no undercut can be formed.

The benzoin-based compounds include, for example, benzoin, benzoin methyl ether, benzoin ethyl ether, benzoin i-propyl ether, benzoin i-butyl ether, methyl-2-benzoin benzoate and the like.

The acetophenone-based compounds include, for example, 2,2-dimethoxy-2-phenylacetophenone, 2-hydroxy-2-methyl-1-phenylpropan-1-one, 1-(4-i-propylphenyl)-2-hydroxy-2-methylpropan-1-one, 4-(2-hydroxyethoxy)phenyl-(2-hydroxy-2-propyl)ketone, 2,2-dimethoxyacetophenone, 2,2-diethoxyacetophenone, 2-methyl-(4-methylthiophenyl)-2-morpholino-1-propan-1-one, 2-benzyl-2-dimethylamino-1-(4-morpholinophenyl)butan-1-one, 1-hydroxycyclohexylphenyl ketone, 4-azidoacetophenone, 4-azidobenzalacetophenone and the like.

The benzophenone-based compounds include, for example, benzophenone, 4,4'-bis(dimethylamino)benzophenone, 4,4'-bis(diethylamino)benzophenone, 3,3'-dimethyl-4-methoxybenzophenone and the like.

The α-diketone-based compounds include, for example, diacetyl, dibenzoyl, methylbenzoyl formate and the like.

The above polynuclear quinone-based compounds include, for example, anthraquinone, 2-ethylanthraquinone, 2-t-butylanthraquinone, 1,4-naphthoquinone and the like.

The xanthone-based compounds include, for example, xanthone, thioxanthone, 2,4-diethylthioxanthone, 2-chlorothioxanthone and the like.

The diazo-based compounds include, for example, 4-diazodiphenylamine, 4-diazo-4'-methoxydiphenylamine, 4-diazo-3-methoxydiphenylamine and the like.

The triazine-based compounds include, for example, 2-(2-furylethylidene)-4,6-bis(trichloromethyl)-s-triazine, 2-(3',4'-dimethoxystyryl)-4,6-bis(trichloromethyl)-s-triazine, 2-(4'-methoxynaphthyl)-4,6-bis(trichloromethyl)-s-triazine, 2-(2'-bromo-4'-methylphenyl)-4,6-bis(trichloromethyl)-s-triazine, 2-(2'-thiophenylethylidene)-4,6-bis(trichloromethyl)-s- triazine and the like.

Photopolymerization initiators other than above compounds include 4-azidobenzaldehyde, azidopyrene, bis(2,6-dimethoxybenzoyl)-2,4,4-trimethylpentylphosphine oxide, N-phenylthioacridone, triphenylpyrylium perchlorate and the like.

In the present invention, the photopolymerization initiators can be used alone or in admixture of two or more.

The amount of the photopolymerization initiator used in the present invention is generally 0.01 to 200 parts by weight, preferably 1 to 120 parts by weight, particularly preferably 1 to 50 parts by weight, based on 100 parts by weight of the polyfunctional monomer (C). When the amount of the photopolymerization initiator is less than 0.01 part by weight, curing by exposure to radiation may be insufficient with the consequence that a pattern may be partially or completely lost or undercut. When the amount is more than 200 parts by weight, the formed pattern easily falls off from the substrate at the time of development, and surface soiling or resin remaining is liable to generate in an area other than portion(s) where the pattern is formed.

Further, in the present invention, a sensitizer, curing promoting agent, polymer photocrosslinking·sensitizing agent and the like can be used in conjunction with the above photopolymerization initiator.

Specific examples of the sensitizer include 4-diethylaminoacetophenone, 4-dimethylaminopropiophenone, ethyl-4-dimethylaminobenzoate, 2-ethylhexyl-1,4-dimethylaminobenzoate, 2,5-bis(4'-diethylaminobenzal)cyclohexanone, 7-diethylamino-3-(4-diethylaminobenzoyl)coumarin, 4-(diethylamino)chalcone and the like.

These sensitizers can be used alone or in admixture of two or more.

Specific examples of the curing promoting agent include chain transfer agents such as 2-mercaptobenzoimidazole, 2-mercaptobenzothiazole, 2-mercaptobenzooxazole, 2,5-dimercapto-1,3,4-thiadiazole, 2-mercapto-4,6-dimethylaminopyridine and the like.

These curing promoting agents can be used alone or in admixture of two or more.

The above polymer photocrosslinking·sensitizing agent is a polymer compound having at least one functional group, which can work as a crosslinking agent and/or sensitizer when exposed to radiation, in a main chain and/or side chain. Specific examples of the photocrosslinking·sensitizing agent include condensates of 4-azidobenzaldehyde and polyvinyl alcohols, condensates of 4-azidobenzaldehyde and phenolic novolak resins, homopolymers and copolymers of 4-acryloylphenylcinnamoyl esters, 1,4-polybutadiene, 1,2-polybutadiene and the like.

These polymer photocrosslinking·sensitiving agents can be used alone or in admixture of two or more.

The total amount of the sensitizer, curing promoting agent and polymer photocrosslinking·sensitizing agent used in the present invention is generally 300 parts or less by weight, preferably 5 to 200 parts by weight, more preferably 10 to 100 parts by weight, based on 100 parts by weight of the photopolymerization initiator.

In the present invention, a combination of a biimidazole-based compound and a benzophenone-based compound as a photopolymerization initiator and/or a thiazole-based curing promoting agent is/are particularly preferably used because formed pixels rarely fall off from the substrate at the time of development and the strength and sensitivity of the pixels are high.

In the present invention, when a biimidazole-based compound is used in combination with other components as a photopolymerization initiator, the total amount of other components used is preferably 80 wt% or less based on the whole photopolymerization initiator.

Examples of combinations of constituent components of the particularly preferred photopolymerization initiator are listed as follows.
2,2'-bis(2-chlorophenyl)-4,4',5,5'-tetrakis(4-ethoxycarbonylphenyl)-1,2'-biimidazole/4,4'-bis(diethylamino)benzophenone,
2,2'-bis(2-chlorophenyl)-4,4',5,5'-tetrakis(4-ethoxycarbonylphenyl)-1,2'-biimidazole/4,4'-bis(diethylamino)benzophenone/2-benzyl-2-dimethylamino-1-(4-morpholinophenyl)butan-1-one,
2,2'-bis(2-chlorophenyl)-4,4',5,5'-tetrakis(4-ethoxycarbonylphenyl)-1,2'-biimidazole/4,4'-bis(diethylamino)benzophenone/1-hydroxycyclohexylphenyl ketone,
2,2'-bis(2-chlorophenyl)-4,4',5,5'-tetrakis(4-ethoxycarbonylphenyl)-1,2'-biimidazole/4,4'-bis(dimethylamino)benzophenone/1-hydroxycyclohexylphenyl ketone/2-mercaptobenzothiazole,
2,2'-bis(2,4-dichlorophenyl)-4,4',5,5'-tetraphenyl-1,2'-biimidazole/4,4'-bis(diethylamino)benzophenone,
2,2'-bis(2,4-dichlorophenyl)-4,4',5,5'-tetraphenyl-1,2'-biimidazole/4,4'-bis(diethylamino)benzophenone/2-benzyl-2-dimethylamino-1-(4-morpholinophenyl)butan-1-one,
2,2'-bis(2,4-dichlorophenyl)-4,4',5,5'-tetraphenyl-1,2'-biimidazole/4,4'-bis(diethylamino)benzophenone/2-benzyl-2-dimethylamino-1-(4-morpholinophenyl)butan-1-one/2-mercaptobenzothiazole,
2,2'-bis(2,4-dibromophenyl)-4,4',5,5'-tetraphenyl-1,2'-biimidazole/4,4'-bis(diethylamino)benzophenone/2-benzyl-2-dimethylamino-1-(4-morpholinophenyl)butan-1-one,
2,2'-bis(2,4-dibromophenyl)-4,4',5,5'-tetraphenyl-1,2'-biimidazole/4,4'-bis(diethylamino)benzophenone/1-hydroxycyclohexylphenyl ketone, and
2,2'-bis(2,4,6-trichlorophenyl)-4,4',5,5'-tetraphenyl-1,2'-biimidazole/4,4'-bis(dimethylamino)benzophenone/1-hydroxycyclohexylphenyl ketone/2-mercaptobenzothiazole.

### Additives

The radiation sensitive composition of the present invention can contain a solvent.

Any solvent is acceptable if it dissolves or disperses the above components (A), (B), (C) and (D) and the above additives to be blended as required, does not react with these components and exhibits appropriate volatility.

Specific examples of the solvent include ethylene glycol monoalkyl ethers such as ethylene glycol monomethyl ether, ethylene glycol monoethyl ether, ethylene glycol mono-n-propyl ether, ethylene glycol mono-n-butyl ether or the like; ethylene glycol monoalkyl ether acetates such as ethylene glycol monomethyl ether acetate, ethylene glycol monoethyl ether acetate or the like; polyethylene glycol monoalkyl ethers such as diethylene glycol monomethyl ether, diethylene glycol monoethyl ether, diethylene glycol mono-n-propyl ether, diethylene glycol mono-n-butyl ether, triethylene glycol monomethyl ether, triethylene glycol monoethyl ether or the like; propylene glycol monoalkyl ethers such as propylene glycol monomethyl ether, propylene glycol monoethyl ether, propylene glycol mono-n-propyl ether, propylene glycol mono-n-butyl ether or the like; polypropylene glycol monoalkyl ethers such as dipropylene glycol monomethyl ether, dipropylene glycol monoethyl ether, dipropylene glycol mono-n-propyl ether, dipropylene glycol mono-n-butyl ether, tripropylene glycol monomethyl ether, tripropylene glycol monoethyl ether or the like; propylene glycol monoalkyl ether acetates such as propylene glycol monomethyl ether acetate, propylene glycol monoethyl ether acetate or the like; other ethers such as diethylene glycol dimethyl ether, diethylene glycol methylethyl ether, diethylene glycol diethyl ether, tetrahydrofuran or the like; ketones such as methyl ethyl ketone, cyclohexanone, 2-heptanone, 3-heptanone or the like; lactic acid alkyl esters such as methyl 2-hydroxypropionate, ethyl 2-hydroxypropionate or the like; other esters such as ethyl 2-methyl-2-hydroxypropionate, methyl 3-methoxypropionate, ethyl 3-methoxypropionate, methyl 3-ethoxypropionate, ethyl 3-ethoxypropionate, ethoxyethyl acetate, ethyl hydroxyacetate, methyl 2-hydroxy-3-methylbutanoate, 3-methyl-3-methoxybutyl acetate, 3-methyl-3-methoxybutyl propionate, ethyl acetate, n-butyl acetate, i-butyl acetate, n-amyl formate, i-amyl acetate, n-butyl propionate, ethyl butyrate, i-propyl butyrate, n-butyl butyrate, methyl pyruvic acid ester, ethyl pyruvic acid ester, n-propyl pyruvic acid ester, acetomethyl acetate, acetoethyl acetate, 2-oxoethylbutanoic acid ester or the like; aromatic hydrocarbons such as toluene, xylene or the like; amides such as N-methylpyrrolidone, N,N-dimethylformamide, N,N-dimethylacetoamide or the like.

These solvents can be used alone or in admixture of two or more.

The solvent can be used in conjunction with a high-boiling solvent such as benzylethyl ether, dihexyl ether, acetonylacetone, isophorone, caproic acid, caprylic acid, 1-octanol, 1-nonanol, benzyl alcohol, benzyl acetate, ethyl benzoate, diethyl oxalate, diethyl maleate, γ-butyrolactone, ethylene carbonate, propylene carbonate or ethylene glycol monophenyl ether acetate.

These high-boiling solvents can be used alone or in admixture of two or more.

Of these, ethylene glycol monomethyl ether acetate, propylene glycol monomethyl ether, propylene glycol monomethyl ether acetate, propylene glycol monoethyl ether acetate, diethylene glycol dimethyl ether, diethylene glycol methylethyl ether, cyclohexanone, 2-heptanone, 3-heptanone, 2-hydroxyethyl propionate, 3-methyl-3-methoxybutyl propionate, 3-methoxyethyl propionate, 3-ethoxymethyl propionate, 3-ethoxyethyl propionate, n-butyl acetate, i-butyl acetate, n-amyl formate, i-amyl acetate, n-butyl propionate, ethyl butylate, i-propyl butylate, n-butyl butylate and ethyl pyruvic acid ester are preferred from the viewpoints of solubility, pigment dispersibility and application properties. Of the above high-boiling solvents, γ-butyrolactone is preferred.

The amount of the solvent used in the present invention is generally 100 to 10,000 parts by weight, preferably 500 to 5,000 parts, based on 100 parts by weight of the alkali-soluble resin (B).

The radiation sensitive composition for use in color filters of the present invention can contain various additives other than a solvent as required.

The additives include, for example, organic acids which serve to improve the solubility in an alkali developer of the radiation sensitive composition and suppress the remaining of undissolved substances of the composition after development.

The organic acids are preferably aliphatic carboxylic acids having a molecular weight of 1,000 or less and phenyl group-containing carboxylic acids having a molecular weight of 1,000 or less.

Specific examples of the aliphatic carboxylic acids include monocarboxylic acids such as formic acid, acetic acid, propionic acid, butyric acid, valeric acid, pivalic acid, caproic acid, diethylacetic acid, enanthic acid, caprylic acid or the like; dicarboxylic acids such as oxalic acid, malonic acid, succinic acid, glutaric acid, adipic acid, pimelic acid, suberic acid, azelaic acid, sebacic acid, brassylic acid, methylmalonic acid, ethylmalonic acid, dimethylmalonic acid, methylsuccinic acid, tetramethylsuccinic acid, cyclohexanedicarboxylic acid, itaconic acid, citraconic acid, maleic acid, fumaric acid, mesaconic acid or the like; and tricarboxylic acids such as tricarballylic acid, aconitic acid, camphoronic acid or the like.

The phenyl group-containing carboxylic acids include, for example, aromatic carboxylic acids having a carboxyl group directly bonded to a phenyl group or carboxylic acids having a carboxyl group bonded to a phenyl group via a carbon chain.

The phenyl group-containing carboxylic acids include, for example, aromatic monocarboxylic acids such as benzoic acid, toluic acid, cuminic acid, hemellitic acid, mesitylenic acid or the like; aromatic dicarboxylic acids such as phthalic acid, isophthalic acid, terephthalic acid or the like; aromatic polycarboxylic acids having a valence of 3 or more such as trimellitic acid, trimesic acid, mellophanic acid, pyromellitic acid or the like; and others such as phenylacetic acid, hydroatropic acid, hydrocinnamic acid, mandelic acid, phenylsuccinic acid, atropic acid, cinnamic acid, cinnamylidenic acid, coumaric acid and umbellic acid.

Of these organic acids, aliphatic dicarboxylic acids and aromatic dicarboxylic acids such as malonic acid, adipic acid, itaconic acid, citraconic acid, fumaric acid, mesaconic acid and phthalic acid are preferred from the viewpoints of alkali solubility, solubility in a solvent and prevention of surface soiling and resin remaining in an area other than portion(s) where pixels are formed, of the substrate.

The organic acids can be used alone or in admixture of two or more.

The amount of the organic acid used is generally 10 wt% or less, preferably 0.001 to 10 wt%, more preferably 0.01 to 1 wt%, based on the whole radiation sensitive composition. When the amount of the organic acid is more than 10 wt%, the adhesion of the formed pixels to the substrate is liable to deteriorate.

Additives other than the above organic acids include, for example, dispersion aids such as blue pigment derivatives and yellow pigment derivative exemplified by copper phthalocyanine derivatives; fillers such as glass and alumina; polymer compounds such as polyvinyl alcohols, polyethylene glycol monoalkyl ethers and poly(fluoroalkylacrylates); nonionic, cationic and anionic surfactants or the like; adhesion promoting agents such as vinyltrimethoxysilane, vinyltriethoxysilane, vinyltris(2-methoxyethoxy)silane, N-(2-aminoethyl)-3-aminopropylmethyldimethoxysilane, N-(2-aminoethyl)-3-aminopropyltrimethoxysilane, 3-aminopropyltriethoxysilane, 3-glycidoxypropyltrimethoxysilane, 3-glycidoxypropylmethyldimethoxysilane, 2-(3,4-epoxycyclohexyl)ethyltrimethoxysilane, 3-chloropropylmethyldimethoxysilane, 3-chloropropyltrimethoxysilane, 3-methacryloxypropyltrimethoxysilane and 3-mercaptopropyltrimethoxysilane; antioxidants such as 2,2-thiobis(4-methyl-6-t-butylphenol) and 2,6-di-t-butylphenol; ultraviolet absorbers such as 2-(3-t-butyl-5-methyl-2-hydroxyphenyl)-5-chlorobenzotriazole and alkoxybenzophenone; agglomeration prevention agents such as sodium polyacrylate; and the like.

### Formation process of color filter

A description is subsequently given of a process for forming a transmission type color filter and a reflection type color filter using the radiation sensitive composition of the present invention.

A light shielding layer for defining a portion having a pixel pattern formed, of the surface of a transparent substrate is first formed, and a composition containing a colorant (A) dispersed therein is applied to the substrate and prebaked to evaporate the solvent to form a coating film. Thereafter, the coating film is exposed to radiation through a photomask, and developed with an alkali developer to dissolve and remove unexposed portions of the coating film, and preferably, post-baked to form an array of colored pixels disposed in a predetermined pattern.

Then, compositions having other colorants (for example, for red, green or blue) dispersed therein are used as required, and arrays of respective colored pixels are formed on the same substrate sequentially by coating the respective compositions, prebaking, exposing to radiation, developing and preferably, post-baking in the same manner as described above. Thus, a color filter is produced.

The transparent substrate used in the formation of the color filter is selected from glass, silicon, polycarbonates, polyesters, aromatic polyamides, polyamideimides, polyimides and the like. These transparent substrates can be subjected, as required, to an appropriate pretreatment such as a chemical treatment with a silane coupling agent or the like, plasma treatment, ion plating, sputtering, gas phase reaction process or vapor deposition.

To apply the radiation sensitive composition for a color filter of the present invention onto the surface of the transparent substrate, a rotation coating, cast coating or roll coating can be suitably employed. The thickness of the coating film is generally 0.1 to 10 µm, preferably 0.2 to 1.5 µm in terms of dried film thickness.

The radiation used to form a color filter is visible light, ultraviolet light, far ultraviolet light, electron beam, X-ray or the like. Radiation having a wavelength of 190 to 450 nm is preferred. The energy of the radiation is preferably 1 to 1,000 mJ/cm².

The alkali developer is preferably an aqueous solution of sodium carbonate, sodium bicarbonate, sodium hydroxide, potassium hydroxide, tetramethylammonium hydroxide, choline, 1,8-diazabicyclo-[5.4.0]-7-undecene, 1,5-diazabicyclo-[4.3.0]-5-nonene or the like.

The alkali developer can contain a water-soluble organic solvent such as methanol or ethanol or a surfactant in a suitable amount. After alkali development, the alkali developer is generally washed away with water.

As the alkali development method, there can be employed a shower development, spray development, dip (immersion) development or paddle development, and the development treatment is preferably carried out at normal temperature for 5 to 300 seconds.

The thus formed transmission type color filter and reflection type color filter are advantageously used in color image pickup tube elements in particular and also useful for color liquid crystal display devices, color sensors and the like.

### Practical Embodiment of the Invention

The radiation sensitive composition of the present invention comprises (A) a colorant, (B) an alkali-soluble resin, (C) a polyfunctional monomer and (D) a photopolymerization initiator as essential ingredients. Particularly preferred examples of the radiation sensitive composition are:
(i) a radiation sensitive composition comprising a carboxyl group-containing copolymer (b) as the component (B) and at least one member selected from the group consisting of 2,2'-bis(2-chlorophenyl)-4,4',5,5'-tetrakis(4-ethoxycarbonylphenyl)-1,2'-biimidazole and 2,2'-bis(2-bromophenyl)-4,4',5,5'-tetrakis(4-ethoxycarbonylphenyl)-1,2'-biimidazole as the component (D);
(ii) a radiation sensitive composition comprising a carboxyl group-containing copolymer (b) as the component (B) and at least one member selected from the group consisting of 2,2'-bis(2,4-dichlorophenyl)-4,4',5,5'-tetraphenyl-1,2'-biimidazole, 2,2'-bis(2,4,6-trichlorophenyl)-4,4',5,5'-tetraphenyl-1,2'-biimidazole, 2,2'-bis(2,4-dibromophenyl)-4,4',5,5'-tetraphenyl-1,2'-biimidazole and 2,2'-bis(2,4,6-tribromophenyl)-4,4',5,5'-tetraphenyl-1,2'-biimidazole as the component (D);
(iii) the radiation sensitive composition (i) or (ii) which further comprises at least one member selected from the group consisting of other photopolymerization initiators, sensitizers, curing promoting agents and polymer photocrosslinking·sensitizing agents as the component (D);
(iv) the radiation sensitive composition (i), (ii) or (iii) which comprises at least one member selected from the group consisting of methacrylic acid/benzyl methacrylate copolymer, methacrylic acid/styrene/methyl methacrylate copolymer, methacrylic acid/styrene/benzyl methacrylate copolymer, methacrylic acid/methyl methacrylate/polystyrene macromonomer copolymer, methacrylic acid/methyl methacrylate/polymethyl methacrylate macromonomer copolymer, methacrylic acid/benzyl methacrylate/polystyrene macromonomer copolymer, methacrylic acid/benzyl methacrylate/polymethyl methacrylate macromonomer copolymer, methacrylic acid/2-hydroxyethyl methacrylate/benzyl methacrylate/polystyrene macromonomer copolymer, methacrylic acid/2-hydroxyethyl methacrylate/benzyl methacrylate/polymethyl methacrylate macromonomer copolymer, methacrylic acid/styrene/benzyl methacrylate/N-phenylmaleimide copolymer, methacrylic acid/styrene/phenyl methacrylate/N-phenylmaleimide copolymer, methacrylic acid/styrene/benzyl methacrylate/N-phenylmaleimide/polystyrene macromonomer copolymer, methacrylic acid/styrene/benzyl methacrylate/N-phenylmaleimide/polymethyl methacrylate macromonomer copolymer, methacrylic acid/styrene/phenyl methacrylate/N-phenylmaleimide/polystyrene macromonomer copolymer, methacrylic acid/styrene/phenyl methacrylate/N-phenylmaleimide/polymethyl methacrylate macromonomer copolymer, methacrylic acid/styrene/2-hydroxyethyl methacrylate/benzyl methacrylate/N-phenylmaleimide/polystyrene macromonomer copolymer, methacrylic acid/styrene/2-hydroxyethyl methacrylate/benzyl methacrylate/N-phenylmaleimide/polymethyl methacrylate macromonomer copolymer, methacrylic acid/styrene/2-hydroxyethyl methacrylate/phenyl methacrylate/N-phenylmaleimide/polystyrene macromonomer copolymer and methacrylic acid/styrene/2-hydroxyethyl methacrylate/phenyl methacrylate/N-phenylmaleimide/polymethyl methacrylate macromonomer copolymer as the carboxyl group-containing copolymer (b) which is the component (B);
(v) the radiation sensitive composition (i), (ii), (iii) or (iv) which comprises at least one member selected from the group consisting of trimethylolpropane triacrylate, pentaerythritol triacrylate, dipentaerythritol pentaacrylate and dipentaerythritol hexaacrylate as the component (C);
(vi) the radiation sensitive composition (i), (ii), (iii), (iv) or (v) which comprises the pigment (1) and/or the pigment (2) and other colorant as the component (A);
(vii) the radiation sensitive composition (vi) which comprises C.I. Pigment Green 7 as the other colorant;
(viii) the radiation sensitive composition (vi) or (vii) which comprises C.I. Pigment Green 36 as the other colorant;
(ix) the radiation sensitive composition (vi), (vii) or (viii) which comprises C.I, Pigment Red 254 as the other colorant;
(x) the radiation sensitive composition (vi), (vii) (viii) or (ix) which comprises C.I. Pigment Red 177 as the other colorant;
(xi) the radiation sensitive composition (vi), (vii) (viii), (ix) or (x) which comprises C.I. Pigment Red 224 as the other colorant;
(xii) a radiation sensitive composition (vi), (vii) (viii), (ix), (x) or (xi) which comprises C.I. Pigment Yellow 83 as the other colorant;
(xiii) a radiation sensitive composition (vi), (vii) (viii), (ix), (x), (xi) or (xii) which comprises C.I. Pigment Yellow 139 as the other colorant;
(xiv) the radiation sensitive composition (i), (ii), (iii), (iv) or (v) which comprises the pigment (3) and other colorant as the component (A);
(xv) the radiation sensitive composition (xiv) which comprises C.I. Pigment Red 224 as the other colorant;
(xvi) the radiation sensitive composition (xiv) or (xv) which comprises C.I. Pigment Red 177 as the other colorant;
(xvii) the radiation sensitive composition (xiv), (xv) or (xvi) which comprises C.I. Pigment Yellow 139 as the other colorant;
(xviii) the radiation sensitive composition (xiv), (xv), (xvi) or (xvii) which comprises C.I. Pigment Yellow 83 as the other colorant;
(xix) the radiation sensitive composition (xiv), (xv), (xvi), (xvii) or (xviii) which comprises the pigment (1) and/or the pigment (2) as the other colorant;
(xx) the radiation sensitive composition (i), (ii), (iii), (iv) or (v) which comprises the pigment (4) and/or the pigment (5) and other colorant as the component (A);
(xxi) the radiation sensitive composition (xix) which comprises C.I. Pigment Red 254 as the other colorant;
(xxii) the radiation sensitive composition (xx) or (xxi) which comprises C.I. Pigment Red 177 as the other colorant;
(xxiii) the radiation sensitive composition (xx), (xxi) or (xxii) which comprises C.I. Pigment Yellow 139 as the other colorant;
(xxiv) the radiation sensitive composition (xx), (xxi), (xxii) or (xxiii) which comprises C.I. Pigment Yellow 83 as the other colorant; and
(xxv) the radiation sensitive composition (xx), (xxi), (xxii), (xxiii) or (xxiv) which comprises the pigment (1) and/or the pigment (2) as the other colorant.

The following examples are provided for the purpose of further illustrating the present invention, but are in no way to be taken as limiting.

### Comparative Example 1

80 Parts by weight of a mixture of C.I. Pigment Yellow 83 and C.I. Pigment Green 36 (weight ratio of 17/83) as the component (A), 50 parts by weight of a methacrylic acid/benzyl methacrylate/polystyrene macromonomer copolymer (weight ratio of 25/65/10, weight-average molecular weight of 55,000) as the component (B), 40 parts by weight of dipentaerythritol pentaacrylate as the component (C), 10 parts by weight of 2,2'-bis(2-chlorophenyl)-4,4',5,5'-tetraphenyl-1,2'-biimidazole and 10 parts by weight of 4,4'-bis(diethylamino)benzophenone as the component (D) and 800 parts by weight of ethylene glycol monoethyl ether acetate as a solvent were mixed together to prepare a composition.

A light shielding layer was then formed on the surface of a transparent soda glass substrate having a silica (SiO₂) film for preventing the elution of sodium ions formed on the surface to define a portion for forming a pixel pattern, and the radiation sensitive composition was applied to the substrate using a spin coater and prebaked at 90°C for 2 minutes to form a 2.0 µm-thick coating film.

Thereafter, after the substrate was cooled, the coating film was exposed to ultraviolet rays having wavelengths of 365 nm, 405 nm and 436 nm and an energy of 100 mJ/cm² through a photomask using a high-pressure mercury lamp. The substrate was then immersed in a 0.1 wt% tetramethyl ammonium hydroxide aqueous solution at 25°C for 1 minute for development, washed with ultra-pure water and dried with air. The substrate was further post-baked at 180°C for 30 minutes to obtain an array of green pixels disposed in a pattern of 20 µm x 20 µm in size.

When the prebaked substrate was observed visually, a swell was seen in a central portion of the substrate. Therefore, coating uniformity was insufficient. When the obtained pixel array was observed through an optical microscope, remaining of undissolved substances of the coating film were observed in an area where the pattern was not formed, of the substrate.

Further, when the Y value in the CIE chromaticity diagram was measured, as an index for the transparency to radiation of the pixel array, at a viewing angle of 2° using a color analyzer (TC-1800M manufactured by Tokyo Denshoku K.K.) with a C light source, it was as low as 55.

### Comparative Example 2

100 Parts by weight of C.I. Pigment Red 177 as the component (A), 50 parts by weight of a methacrylic acid/benzyl methacrylate/polystyrene macromonomer copolymer (weight ratio of 25/65/10, weight-average molecular weight of 55,000) as the component (B), 40 parts by weight of dipentaerythritol pentaacrylate as the component (C), 10 parts by weight of 2,2'-bis(2-chlorophenyl)-4,4',5,5'-tetraphenyl-1,2'-biimidazole and 10 parts by weight of 4,4'-bis(diethylamino)benzophenone as the component (D) and 800 parts by weight of ethylene glycol monoethylether acetate as a solvent were mixed together to prepare a composition.

A light shielding layer was then formed on the surface of a transparent soda glass substrate having a silica (SiO₂) film for preventing the elution of sodium ions formed on the surface to define a portion for forming a pixel pattern, and the radiation sensitive composition was applied to the substrate using a spin coater and prebaked at 90°C for 2 minutes to form a 2.0 µm-thick coating film.

Thereafter, after the substrate was cooled, the coating film was exposed to ultraviolet rays having wavelengths of 365 nm, 405 nm and 436 nm and an energy of 100 mJ/cm² through a photomask using a high-pressure mercury lamp. The substrate was then immersed in a 0.1 wt% tetramethl ammonium hydroxide aqueous solution at 25°C for 1 minute for development, washed with ultra-pure water and dried with air. The substrate was further post-baked at 180°C for 30 minutes to obtain an array of red pixels disposed in a pattern of 20 µm x 20 µm in size.

When the prebaked substrate was observed visually, a swell was seen in a central portion of the substrate. Therefore, coating uniformity was insufficient. When the obtained pixel array was observed through an optical microscope, remaining of undissolved substances of the coating film were observed in an area where the pattern was not formed, of the substrate.

Further, when the Y value in the CIE chromaticity diagram was measured, as an index for the transparency to radiation of the pixel array, at a viewing angle of 2° using a color analyzer (TC-1800M manufactured by Tokyo Denshoku K.K.) with a C light source, it was as low as 20.

### Comparative Example 3

100 Parts by weight of a mixture of C.I. Pigment Red 177 and C.I. Pigment Yellow 139 (weight ratio of 80/20) as the component (A), 50 parts by weight of a methacrylic acid/benzyl methacrylate/polystyrene macromonomer copolymer (weight ratio of 25/65/10, weight-average molecular weight of 55,000) as the component (B), 40 parts by weight of dipentaerythritol pentaacrylate as the component (C), 10 parts by weight of 2,2'-bis(2-chlorophenyl)-4,4',5,5'-tetraphenyl-1,2'-biimidazole and 10 parts by weight of 4,4'-bis(diethylamino)benzophenone as the component (D), 5 parts by weight of malonic acid as an organic acid and 800 parts by weight of ethylene glycol monoethylether acetate as a solvent were mixed together to prepare a composition.

A light shielding layer was then formed on the surface of a transparent soda glass substrate having a silica (SiO₂) film for preventing the elution of sodium ions formed on the surface to define a portion for forming a pixel pattern, and the radiation sensitive composition was applied to the substrate using a spin coater and prebaked at 90°C for 2 minutes to form a 2.0 µm-thick coating film.

Thereafter, after the substrate was cooled, the coating film was exposed to ultraviolet rays having wavelengths of 365 nm, 405 nm and 436 nm and an energy of 100 mJ/cm² through a photomask using a high-pressure mercury lamp. The substrate was then immersed in a 0.1 wt% tetramethl ammonium hydroxide aqueous solution at 25°C for 1 minute for development, washed with ultra-pure water and dried with air. The substrate was further post-baked at 180°C for 30 minutes to produce an array of red pixels disposed in a pattern of 20 µm x 20 µm in size.

When the prebaked substrate was observed visually, a swell was seen in a central portion of the substrate. Therefore, coating uniformity was insufficient. When the obtained pixel array was observed through an optical microscope, remaining of undissolved substances of the coating film were observed in an area where the pattern was not formed, of the substrate.

Further, when the Y value in the CIE chromaticity diagram was measured, as an index for the transparency to radiation of the pixel array, at a viewing angle of 2° using a color analyzer (TC-1800M manufactured by Tokyo Denshoku K.K.) with a C light source, it was as low as 20.

### Comparative Example 4

100 Parts by weight of a mixture of C.I. Pigment Red 177 and C.I. Pigment Yellow 83 (weight ratio of 75/25) as the component (A), 50 parts by weight of a methacrylic acid/benzyl methacrylate/polystyrene macromonomer copolymer (weight ratio of 25/65/10, weight-average molecular weight of 55,000) as the component (B), 40 parts by weight of dipentaerythritol pentaacrylate as the component (C), 10 parts by weight of 2,2'-bis(2-chlorophenyl)-4,4',5,5'-tetraphenyl-1,2'-biimidazole and 10 parts by weight of 4,4'-bis(diethylamino)benzophenone as the component (D), 5 parts by weight of citraconic acid as an organic acid and 800 parts by weight of ethylene glycol monoethylether acetate as a solvent were mixed together to prepare a composition.

A light shielding layer was then formed on the surface of a transparent soda glass substrate having a silica (SiO₂) film for preventing the elution of sodium ions formed on the surface to define a portion for forming a pixel pattern, and the radiation sensitive composition was applied to the substrate using a spin coater and prebaked at 90°C for 2 minutes to form a 2.0 µm-thick coating film.

Thereafter, after the substrate was cooled, the coating film was exposed to ultraviolet rays having wavelengths of 365 nm, 405 nm and 436 nm and an energy of 100 mJ/cm² through a photomask using a high-pressure mercury lamp. The substrate was then immersed in a 0.1 wt% tetramethl ammonium hydroxide aqueous solution at 25°C for 1 minute for development, washed with ultra-pure water and dried with air. The substrate was further post-baked at 180°C for 30 minutes to obtain an array of red pixels disposed in a pattern of 20 µm x 20 µm in size.

When the prebaked substrate was observed visually, a swell was seen in a central portion of the substrate. Therefore, coating uniformity was insufficient. When the obtained pixel array was observed through an optical microscope, remaining of undissolved substances of the coating film were observed in an area where the pattern was not formed, of the substrate.

Further, when the Y value in the CIE chromaticity diagram was measured, as an index for the transparency to radiation of the pixel array, at a viewing angle of 2° using a color analyzer (TC-1800M manufactured by Tokyo Denshoku K.K.) with a C light source, it was as low as 21.

### Example 1

80 Parts by weight of a mixture of C.I. Pigment Yellow 150 and C.I. Pigment Green 36 (weight ratio of 35/65) as the component (A), 50 parts by weight of a methacrylic acid/benzyl methacrylate/polystyrene macromonomer copolymer (weight ratio of 25/65/10, weight-average molecular weight of 55,000) as the component (B), 40 parts by weight of dipentaerythritol pentaacrylate as the component (C), 10 parts by weight of 2,2'-bis(2-chlorophenyl)-4,4',5,5'-tetraphenyl-1,2'-biimidazole and 10 parts by weight of 4,4'-bis(diethylamino)benzophenone as the component (D) and 800 parts by weight of ethylene glycol monoethylether acetate as a solvent were mixed together to prepare a composition.

An array of green pixels disposed in a pattern was prepared using the above composition in the same manner as in Comparative Example 1. The thickness of the coating film was adjusted to obtain the same chromaticity (x and y values in the CIE chromaticity diagram) as that of Comparative Example 1.

When the prebaked substrate was observed visually, no swell was seen in a central portion of the substrate. Therefore, coating uniformity was excellent. When the obtained pixel array was observed through an optical microscope, no remaining of undissolved substances of the coating film were observed in an area where the pattern was not formed. Therefore, developability was excellent and the shape of the pattern was satisfactory.

Further, when the Y value in the CIE chromaticity diagram was measured, as an index for the transparency to radiation of the pixel array, at a viewing angle of 2° using a color analyzer (TC-1800M manufactured by Tokyo Denshoku K.K.) With a C light source, it was as high as 63. The chroma and lightness of the obtained pixel array were well-balanced.

### Example 2

80 Parts by weight of a mixture of C.I. Pigment Yellow 155 and C.I. Pigment Green 36 (weight ratio of 35/65) as the component (A), 50 parts by weight of a methacrylic acid/benzyl methacrylate/polystyrene macromonomer copolymer (weight ratio of 25/65/10, weight-average molecular weight of 55,000) as the component (B), 40 parts by weight of dipentaerythritol pentaacrylate as the component (C), 10 parts by weight of 2,2'-bis(2-chlorophenyl)-4,4',5,5'-tetraphenyl-1,2'-biimidazole and 10 parts by weight of 4,4'-bis(diethylamino)benzophenone as the component (D) and 800 parts by weight of ethylene glycol monoethylether acetate as a solvent were mixed together to prepare a composition.

An array of green pixels disposed in a pattern was prepared using the above composition in the same manner as in Comparative Example 1. The thickness of the coating film was adjusted to obtain the same chromaticity (x and y values in the CIE chromaticity diagram) as that of Comparative Example 1.

When the prebaked substrate was observed visually, no swell was seen in a central portion of the substrate. Therefore, coating uniformity was excellent. When the obtained pixel array was observed through an optical microscope, no remaining of undissolved substances of the coating film were observed in an area where the pattern was not formed. Therefore, developability was excellent and the shape of the pattern was satisfactory.

Further, when the Y value in the CIE chromaticity diagram was measured, as an index for the transparency to radiation of the pixel array, at a viewing angle of 2° using a color analyzer (TC-1800M manufactured by Tokyo Denshoku K.K.) with a C light source, it was as high as 63. The chroma and lightness of the obtained pixel array were well-balanced.

### Example 3

80 Parts by weight of a mixture of C.I. Pigment Yellow 150, C.I. Pigment Yellow 155 and C.I. Pigment Green 36 (weight ratio of 17.5/17.5/65) as the component (A), 50 parts by weight of a methacrylic acid/benzyl methacrylate/polystyrene macromonomer copolymer (weight ratio of 25/65/10, weight-average molecular weight of 55,000) as the component (B), 40 parts by weight of dipentaerythritol pentaacrylate as the component (C), 10 parts by weight of 2,2'-bis(2-chlorophenyl)-4,4',5,5'-tetraphenyl-1,2'-biimidazole and 10 parts by weight of 4,4'-bis(diethylamino)benzophenone as the component (D) and 800 parts by weight of ethylene glycol monoethylether acetate as a solvent were mixed together to prepare a composition.

An array of green pixels disposed in a pattern was prepared using the above composition in the same manner as in Comparative Example 1. The thickness of the coating film was adjusted to obtain the same chromaticity (x and y values in the CIE chromaticity diagram) as that of Comparative Example 1.

When the prebaked substrate was observed visually, no swell was seen in a central portion of the substrate. Therefore, coating uniformity was excellent. When the obtained pixel array was observed through an optical microscope, no remaining of undissolved substances of the coating film were observed in an area where the pattern was not formed. Therefore, developability was excellent and the shape of the pattern was satisfactory.

Further, when the Y value in the CIE chromaticity diagram was measured, as an index for the transparency to radiation of the pixel array, at a viewing angle of 2° using a color analyzer (TC-1800M manufactured by Tokyo Denshoku K.K.) with a C light source, it was as high as 63. The chroma and lightness of the obtained pixel array were well-balanced.

### Example 4

100 Parts by weight of C.I. Pigment Red 254 as the component (A), 50 parts by weight of a methacrylic acid/benzyl methacrylate/polystyrene macromonomer copolymer (weight ratio of 25/65/10, weight-average molecular weight of 55,000) as the component (B), 40 parts by weight of dipentaerythritol pentaacrylate as the component (C), 10 parts by weight of 2,2'-bis(2-chlorophenyl)-4,4',5,5'-tetraphenyl-1,2'-biimidazole and 10 parts by weight of 4,4'-bis(diethylamino)benzophenone as the component (D) and 800 parts by weight of ethylene glycol monoethylether acetate as a solvent were mixed together to prepare a composition.

An array of red pixels disposed in a pattern on the substrate was prepared using the above composition in the same manner as in Comparative Example 2. The thickness of the coating film was adjusted to obtain the same chromaticity (x and y values in the CIE chromaticity diagram) as that of Comparative Example 2.

When the prebaked substrate was observed visually, no swell was seen in a central portion of the substrate. Therefore, coating uniformity was excellent. When the obtained pixel array was observed through an optical microscope, no remaining of undissolved substances of the coating film were observed in an area where the pattern was not formed. Therefore, developability was excellent and the shape of the pattern was satisfactory.

Further, when the Y value in the CIE chromaticity diagram was measured, as an index for the transparency to radiation of the pixel array, at a viewing angle of 2° using a color analyzer (TC-1800M manufactured by Tokyo Denshoku K.K.) with a C light source, it was as high as 24. The chroma and lightness of the obtained pixel array were well-balanced.

### Example 5

80 Parts by weight of a mixture of C.I. Pigment Red 224, C.I. Pigment Red 177 and C.I. Pigment Yellow 139 (weight ratio 47/49/4) as the component (A), 50 parts by weight of a methacrylic acid/benzyl methacrylate/polystyrene macromonomer copolymer (weight ratio of 25/65/10, weight-average molecular weight of 55,000) as the component (B), 40 parts by weight of dipentaerythritol pentaacrylate as the component (C), 10 parts by weight of 2,2'-bis(2-chlorophenyl)-4,4',5,5'-tetraphenyl-1,2'-biimidazole and 10 parts by weight of 4,4'-bis(diethylamino)benzophenone as the component (D), 5 parts by weight of malonic acid as an organic acid and 800 parts by weight of ethylene glycol monoethylether acetate as a solvent were mixed together to prepare a composition.

A light shielding layer was then formed on the surface of a transparent soda glass substrate having a silica (SiO₂) film for preventing the elution of sodium ions formed on the surface to define a portion for forming a pixel pattern, and the radiation sensitive composition was applied to the substrate using a spin coater and prebaked at 90°C for 2 minutes to form a coating film.

Thereafter, after the substrate was cooled, the coating film was exposed to ultraviolet rays having wavelengths of 365 nm, 405 nm and 436 nm and an energy of 100 mJ/cm² through a photomask using a high-pressure mercury lamp. The substrate was then immersed in a 0.1 wt% tetramethl ammonium hydroxide aqueous solution at 25°C for 1 minute for development, washed with ultra-pure water and dried with air. The substrate was further post-baked at 180°C for 30 minutes to produce an array of red pixels disposed in a pattern of 20 µm x 20 µm in size. The thickness of the coating film was adjusted to obtain the same chromaticity (x and y values in the CIE chromaticity diagram) as that of Comparative Example 3.

When the prebaked substrate was observed visually, no swell was seen in a central portion of the substrate. Therefore, coating uniformity was excellent. When the obtained pixel array was observed through an optical microscope, no remaining of undissolved substances of the coating film were observed in an area where the pattern was not formed. Therefore, developability was excellent and the shape of the pattern was satisfactory.

Further, when the Y value in the CIE chromaticity diagram was measured, as an index for the transparency to radiation of the pixel array, at a viewing angle of 2° using a color analyzer (TC-1800M manufactured by Tokyo Denshoku K.K.) with a C light source, it was as high as 24. The chroma and lightness of the obtained pixel array were well-balanced.

### Example 6

100 Parts by weight of C.I. Pigment Red 224 as the component (A), 50 parts by weight of a methacrylic acid/benzyl methacrylate/polystyrene macromonomer copolymer (weight ratio of 25/65/10, weight-average molecular weight of 55,000) as the component (B), 40 parts by weight of dipentaerythritol pentaacrylate as the component (C), 10 parts by weight of 2,2'-bis(2-chlorophenyl)-4,4',5,5'-tetraphenyl-1,2'-biimidazole and 10 parts by weight of 4,4'-bis(diethylamino)benzophenone as the component (D), 5 parts by weight of citraconic acid as an organic acid and 800 parts by weight of ethylene glycol monoethylether acetate as a solvent were mixed together to prepare a composition.

A light shielding layer was then formed on the surface of a transparent soda glass substrate having a silica (SiO₂) film for preventing the elution of sodium ions formed on the surface to define a portion for forming a pixel pattern, and the radiation sensitive composition was applied to the substrate using a spin coater and prebaked at 90°C for 2 minutes to form a coating film.

Thereafter, after the substrate was cooled, the coating film was exposed to ultraviolet rays having wavelengths of 365 nm, 405 nm and 436 nm and an energy of 100 mJ/cm² through a photomask using a high-pressure mercury lamp. The substrate was then immersed in a 0.1 wt% tetramethl ammonium hydroxide aqueous solution at 25°C for 1 minute for development, washed with ultra-pure water and dried with air. The substrate was further post-baked at 180°C for 30 minutes to produce an array of red pixels disposed in a pattern of 20 µm x 20 µm in size.

When the prebaked substrate was observed visually, no swell was seen in a central portion of the substrate. Therefore, coating uniformity was excellent. When the obtained pixel array was observed through an optical microscope, no remaining of undissolved substances of the coating film were observed in an area where the pattern was not formed. Therefore, developability was excellent and the shape of the pattern was satisfactory.

Further, when the Y value in the CIE chromaticity diagram was measured, as an index for the transparency to radiation of the pixel array, at a viewing angle of 2° using a color analyzer (TC-1800M manufactured by Tokyo Denshoku K.K.) with a C light source, it was as high as 27. The chroma and lightness of the obtained pixel array were well-balanced.

The radiation sensitive composition for color filters, provided by the present invention, is excellent in coating uniformity, developability and sensitivity for the formation of a pixel array, and can form a pixel array having a good pattern shape, excellent transparency to radiation and good balance between chroma and lightness.

A radiation sensitive composition containing (A) at least one colorant selected from an azo pigment, a cyclic diketone pigment and a perylene pigment, (B) an alkali-soluble resin, (C) a polyfunctional monomer and (D) a photopolymerization initiator.

## Claims

1. A radiation sensitive composition comprising:
(A) at least one colorant selected from the group consisting of an azo pigment represented by the following formula (1) and a metal complex thereof: 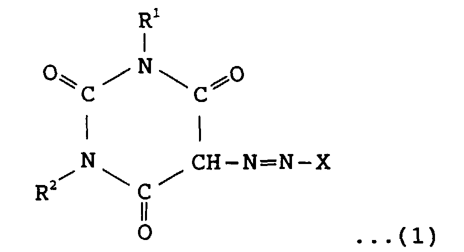
wherein X is a substituted or unsubstituted monovalent aromatic hydrocarbon group having 6 to 20 carbon atoms, or a group having a substituted or unsubstituted monovalent 5- or 6-membered nitrogen-containing heterocyclic group, and R¹ and R² are independently a hydrogen atom, or a substituted or unsubstituted monovalent hydrocarbon group having 1 to 5 carbon atoms,
an azo pigment represented by the following formula (2): 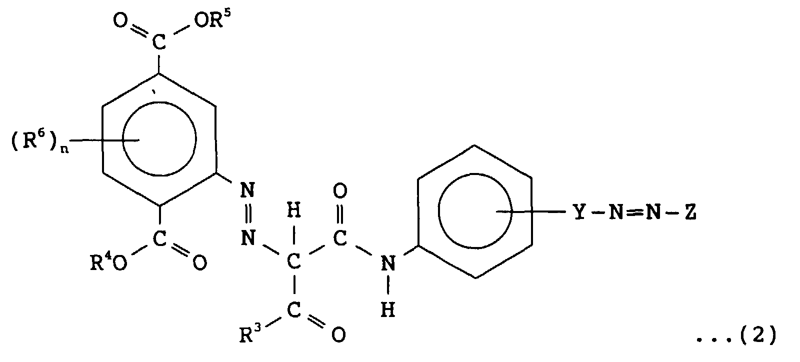
wherein Y is a divalent bond, Z is a substituted or unsubstituted monovalent aromatic hydrocarbon group having 6 to 20 carbon atoms, or a group having a substituted or unsubstituted monovalent 5- or 6-membered nitrogen-containing heterocyclic group, R³, R⁴ and R⁵ are independently a hydrogen atom, or a substituted or unsubstituted monovalent hydrocarbon group having 1 to 5 carbon atoms, R⁶ is a monovalent substituent group, and n is an integer of 0 to 3,
a cyclic diketone compound pigment, a perylene pigment represented by the following formula (3): 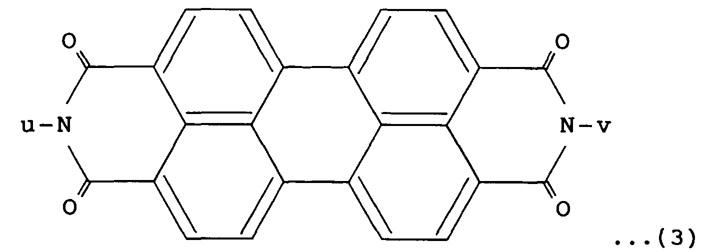
wherein u and v are independently an alkyl group having 1 to 10 carbon atoms, a substituted or unsubstituted monovalent aromatic hydrocarbon group having 6 to 20 carbon atoms, or a group having a substituted or unsubstituted monovalent 5- or 6-membered nitrogen-containing heterocyclic group,
and a perylene pigment represented by the following formula (4): 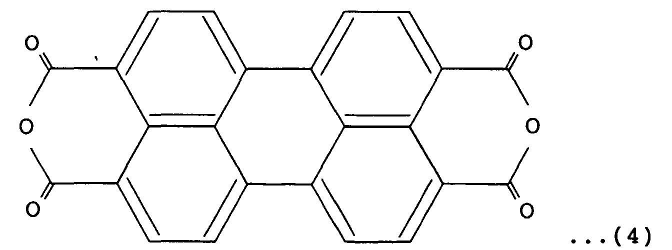
(B) an alkali-soluble resin,
(C) a polyfunctional monomer, and
(D) a photopolymerization initiator.

2. The radiation sensitive composition of claim 1, wherein in the formula (1) representing the colorant, X is a substituted or unsubstituted monovalent aromatic hydrocarbon group having 6 to 20 carbon atoms, and the aromatic hydrocarbon group is phenyl group, 4-biphenyl group, 1-naphthyl group, 2-naphthyl group, 1-anthranyl group, 2-anthranyl group, 9-anthranyl group or 9-phenanthryl group.

3. The radiation sensitive composition of claim 2, wherein the substituent group is a halogen atom, nitro group, cyano group, or group represented by -OR⁷, -COR⁸, -COOR⁹, -N(R¹⁰)(R¹¹), -SO₃R¹², -SO₂R¹³, or -OCOR¹⁴ (in which R⁷ to R¹² are independently a hydrogen atom, alkyl group having 1 to 5 carbon atoms, aryl group having 6 to 12 carbon atoms or aralkyl group having 7 to 13 carbon atoms, and R¹³ and R¹⁴ are independently an alkyl group having 1 to 5 carbon atoms, aryl group having 6 to 12 carbon atoms or aralkyl group having 7 to 13 carbon atoms).

4. The radiation sensitive composition of claim 1, wherein in the formula (1) representing the colorant, X is a group having a substituted or unsubstituted monovalent 5- or 6-membered nitrogen-containing heterocyclic group, and the nitrogen-containing heterocyclic group is selected from the group consisting of pyrrolyl group, pyrazolyl group, imidazolyl group, triazolyl group, pyridinyl group, pyrimidinyl group, pyrazinyl group, 1,3,5-triazinyl group, oxazolyl group, isooxazolyl group, thiazolyl group, isothiazolyl group and groups represented by the following formula (5) to (8).

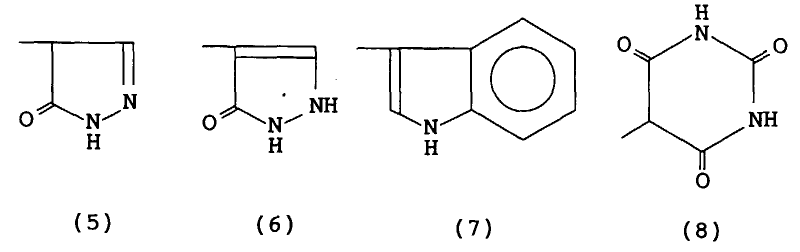

5. The radiation sensitive composition of claim 4, wherein the substituent group is a group selected from the group consisting of an alkyl group having 1 to 5 carbon atoms, aryl group having 6 to 12 carbon atoms and aralkyl group having 7 to 13 carbon atoms that is present on the nitrogen atom of the nitrogen-containing heterocyclic group, or a group selected from the group consisting of an alkyl group having 1 to 5 carbon atoms, aryl group having 6 to 12 carbon atoms, aralkyl group having 7 to 13 carbon atoms and substituent groups specified in claim 3 that are present on the carbon atom of the nitrogen-containing heterocyclic group.

6. The radiation sensitive composition of claim 1, wherein in the formula (1) representing the colorant, R¹ and R² are independently a substituted or unsubstituted monovalent hydrocarbon group having 1 to 5 carbon atoms, and the hydrocarbon group is selected from the group consisting of methyl group, ethyl group, n-propyl group, i-propyl group, n-butyl group, i-butyl group, sec-butyl group, t-butyl group and n-pentyl group.

7. The radiation sensitive composition of claim 6, wherein the substituent group is selected from the group consisting of substituent groups specified in claim 3.

8. The radiation sensitive composition of claim 1, wherein the metal of the metal complex of the azo pigment represented by the formula (1) is selected from the group consisting of iron, cobalt, nickel, chromium, copper and magnesium.

9. The radiation sensitive composition of claim 1, wherein in the formula (2) representing the colorant, Y is selected from the group consisting of a single bond, alkylene group having 1 to 5 carbon atoms, arylene group having 6 to 12 carbon atoms, aralkylene group having 7 to 13 carbon atoms and groups represented by -O-(CH₂)ᵢ-, -S-(CH₂)ᵢ-, -CO-(CH₂)ᵢ-, -COO-(CH₂)ᵢ-, -OCO-(CH₂)ᵢ-, -SO₂-(CH₂)ᵢ-, -NH-(CH₂)ᵢ-, -NHCO-(CH₂)ᵢ- and 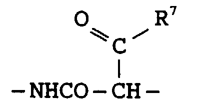 (in which i is an integer of 1 to 5, and R⁷ is a hydrogen atom, or a substituted or unsubstituted monovalent hydrocarbon group having 1 to 5 carbon atoms).

10. The radiation sensitive composition of claim 1, wherein in the formula (2) representing the colorant, Z is a substituted or unsubstituted monovalent aromatic hydrocarbon group having 6 to 20 carbon atoms, and the aromatic hydrocarbon group is selected from the group consisting of phenyl group, biphenyl group, 1-naphthyl group, 2-naphthyl group, 1-anthranyl group, 2-anthranyl group, 9-anthranyl group and 9-phenanthryl group.

11. The radiation sensitive composition of claim 10, wherein the substituent group is selected from the group consisting of substituent groups specified in claim 3.

12. The radiation sensitive composition of claim 1, wherein in the formula (2) representing the colorant, Z is a group having a substituted or unsubstituted nitrogen-containing heterocyclic group, and the nitrogen-containing heterocyclic group is selected from the group consisting of pyrrolyl group, pyrazolyl group, imidazolyl group, triazolyl group, pyridinyl group, pyrimidinyl group, pyrazinyl group, 1,3,5-triazinyl group, oxazolyl group, isooxazolyl group, thiazolyl group, isothiazolyl group, and groups represented by the aforesaid formulas (5) to (8).

13. The radiation sensitive composition of claim 12, wherein the substituent group is selected from the group consisting of substituent groups specified in claim 5.

14. The radiation sensitive composition of claim 1, wherein in the formula (2) representing the colorant, R³, R⁴ and R⁵ are independently a substituted or unsubstituted monovalent hydrocarbon group having 1 to 5 carbon atoms, and the hydrocarbon group is selected from the group consisting of methyl group, ethyl group, n-propyl group, i-propyl group, n-butyl group, i-butyl group, sec-butyl group, t-butyl group and n-pentyl group.

15. The radiation sensitive composition of claim 14, wherein the substituent group is selected from the group consisting of substituent groups specified in claim 3.

16. The radiation sensitive composition of claim 1, wherein in the formula (2) representing the colorant, R⁶ is a substituent group selected from the group consisting of substituent groups specified in claim 3.

17. The radiation sensitive composition of claim 1, wherein in the formula (3) representing the colorant, u and v are independently an alkyl group having 1 to 10 carbon atoms, and the alkyl group is selected from the group consisting of methyl group, ethyl group, n-propyl group, i-propyl group, n-butyl group, i-butyl group, sec-butyl group, t-butyl group, n-pentyl group, n-hexyl group, n-heptyl group, n-octyl group, 2-ethylhexyl group, n-nonyl group and n-decyl group.

18. The radiation sensitive composition of claim 1, wherein in the formula (3) representing the colorant, u and v are independently a substituted or unsubstituted monovalent aromatic hydrocarbon group, and the aromatic hydrocarbon group is selected from the group consisting of phenyl group, 4-biphenyl group, 1-naphthyl group, 2-naphthyl group, 1-anthranyl group, 2-anthranyl group, 9-anthranyl group and 9-phenanthryl group.

19. The radiation sensitive composition of claim 18, wherein the substituent group is selected from the group consisting of substituent groups specified in claim 3.

20. The radiation sensitive composition of claim 1, wherein in the formula (3) representing the colorant, u and v are independently a group having a substituted or unsubstituted monovalent 5- or 6-membered nitrogen-containing heterocyclic group, and the nitrogen-containing heterocyclic group is selected from the group consisting of 1-pyrrolyl group, 2-pyrrolyl group, 3-pyrrolyl group, 1-pyrazolyl group, 3-pyrazolyl group, 4-pyrazolyl group, 1-imidazolyl group, 2-imidazolyl group, 4-imidazolyl group, 1-triazolyl group, 4-triazolyl group, 5-triazolyl group, 2-pyridinyl group, 3-pyridinyl group, 4-pyridinyl group, 2-pyrimidinyl group, 4-pyrimidinyl group, 5-pyrimidinyl group, 6-pyrimidinyl group, 2-pyrazinyl group, 3-pyrazinyl group, s-triazinyl group, 2-oxazolyl group, 4-oxazolyl group, 5-oxazolyl group, 3-isooxazolyl group, 4-isooxazolyl group, 5-isooxazolyl group, 2-thiazolyl group, 4-thiazolyl group, 5-thiazolyl group, 3-isothiazolyl group, 4-isothiazolyl group and 5-isothiazolyl group.

21. The radiation sensitive composition of claim 20, wherein the substituent group is selected from the group consisting of an alkyl group having 1 to 5 carbon atoms, aryl group having 6 to 12 carbon atoms and aralkyl group having 7 to 13 carbon atoms.

22. The radiation sensitive composition of claim 1, which further comprises another colorant different from the colorant (A).

23. The radiation sensitive composition of claim 1, wherein the alkali-soluble resin (B) is selected from the group consisting of a copolymer of an ethylenically unsaturated monomer having at least one carboxyl group in the molecule and another ethylenically unsaturated monomer copolymerizable with the monomer, alkali-soluble polyester resin and alkali-soluble phenolic resin.

24. The radiation sensitive composition of claim 1 which contains the alkali-soluble resin (B) in an amount of 10 to 1,000 parts by weight based on 100 parts by weight of the colorant (A).

25. The radiation sensitive composition of claim 1, wherein the polyfunctional monomer (C) is a poly(meth)acrylate of a polyhydric alcohol having at least three hydroxyl groups.

26. The radiation sensitive composition of claim 1 which contains the polyfunctional monomer (C) in an amount of 5 to 500 parts by weight based on 100 parts by weight of the alkali-soluble resin (B).

27. The radiation sensitive composition of claim 1 which contains the photopolymerization initiator (D) in an amount of 0.01 to 200 parts by weight based on 100 parts by weight of the polyfunctional monomer (C).
